# EUROPEAN PATENT APPLICATION

(11) **EP 2 730 558 A1**
(43) Date of publication of application: **14.05.2014**
(21) Application number: 12382437.7
(22) Date of filing: 08.11.2012
(51) Int. Cl.: C07C 225/22, C07D 209/04, C07D 209/12, A61K 31/404, A61P 35/00

(54) **Indole derivatives, pharmaceutical compositions containing such indoles and their use as DNA methylation modulators**

(71) Applicant: Ikerchem, S.L., 20009 Donostia - San Sebastián (ES); Universidad del Pais Vasco, 48940 Leioa Vizcaya (ES)
(72) Inventor: Cossío Mora, Fernando Pedro, E-20018 San Sebastian - Guipúzcoa (ES); Vara Salazar, Yosu Ion, E-20009 San Sebastián - Guipúzcoa (ES); Masdeu Margalef, María del Carmen, E-20009 San Sebastián - Guipúzcoa (ES); Alcalá Caffarena, María Remedios, E-20009 San Sebastián - Guipúzcoa (ES); Villafruela Cáneva, Sergio, E-20009 San Sebastián - Guipúzcoa (ES); Otaegui Ansa, Dorleta, E-20009 San Sebastián - Guipúzcoa (ES); Aldaba Arévalo, Eneko, E-20009 San Sebastián - Guipúzcoa (ES); San Sebastián Larzabal, Eider, E-20009 San Sebastián - Guipúzcoa (ES); Zubia Olascoaga, Aizpea, E-20009 San Sebastián - Guipúzcoa (ES)
(74) Representative: ABG Patentes, S.L.

(57) **Abstract**

The present invention refers to compounds of formula (I): 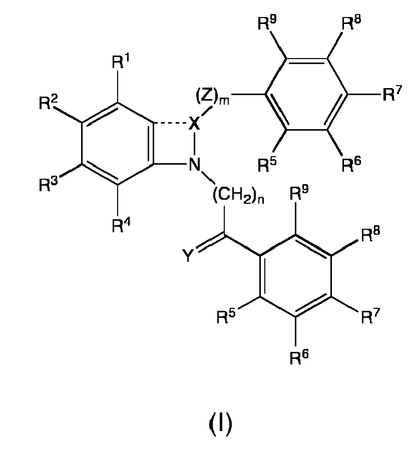 as well as to a method for their preparation, pharmaceutical compositions comprising the same, and use thereof for the treatment and/or chemoprevention of cancer hematological malignancy, proliferative diseases, genetic diseases, neurological disorders and immunological disorders.

## Description

### FIELD OF THE INVENTION

The present invention is related to new compounds derived from polysubstituted indole rings, processes for their preparation, pharmaceutical compositions containing such compounds and use thereof as inhibitors of DNA methylation and therapeutic agents for preventing or treating diseases associated with aberrant DNA methylation such as cancer, hematological malignancy, proliferative diseases, genetic diseases, neurological disorders and immunological disorders.

### BACKGROUND OF THE INVENTION

DNA methyltransferase (DNMT) enzymes promote the covalent addition of a methyl group to a specific nucleotide base in DNA, using S-adenosyl methionine (SAM) as the methyl donor. Three DNMT enzymes are involved in the control of the methylation state of the C-5 position of cytosine residues located at CpG dinucleotides in genome: DNMT1, DNMT3A and DNMT3B. By specifically inhibiting DNMTs, the aberrant methylation of the DNA gene promoter regions can be prevented. Thus, DNMT enzymes constitute a therapeutic target for preventing or treating diseases associated with aberrant DNA methylation.

Hypermethylation of DNA gene promoter regions is the cause of a number of inherited disease syndromes, and can also have a major role in the development of human cancer (cf. F. Gaudet et al. Science 2003, 300, 489*;* A. Eden et al. Science 2003, 300, 455). It is the most frequent molecular change in hematopoietic neoplasms (cf. G. Egger et al. Nature 2004, 429, 457) and is likely involved in other tumor types, such as colorectal cancer (cf. M. F. Kane et al. Cancer Res. 1997, 57, 808) and prostate cancer (cf. S. Yegnasubramanian et al. Cancer Res. 2004, 64, 1975; C. Jeronimo et al. Clin. Cancer Res. 2004, 10, 8472; G. H. Kang et al. J. Pathol. 2004, 202, 233; M. Sasaki et al. J. Natl Cancer Inst. 2002, 94, 384).

Dysregulation of epigenetic marks or epigenetic mechanisms related to DNMT function have been recognized to occur also in other diseases and syndromes (cf. "Epigenetics in Biology and Medicine", edited by M. Esteller, 1st Edition (2008), CRC Press Inc), which include genetic syndromes such as ATR-X, Rett, Fragile X, Prader-Willi, Angelman, CHARGE, CSB, SIOD (Schimke Immuno-Osseous Dysplasia), ICF, Rubinstein-Taybi syndrome and FSHD (Facioscapulo-humeral Dystrophy). Also, immune diseases such as systemic lupus erythematosus (SLE), rheumatoid arthritis or progressive systemic sclerosis (PSS), as well as neurologic disorders such as schizophrenia and Alzheimer (cf. J. Gräff, I.M. Mansuy Behav. Brain Res. 2008, 192, 70; J. Gräff, I.M. Mansuy Eur J Neurosci 2009, 30, 1) show disregulation of DNMT function.

On the other hand, 1*H*-indoles have been included in the category of "privileged structures" (cf. D. A. Horton, G. T. Bourne and M. L. Smythe Chem. Rev. 2003, 103, 893; D. Müller Drug Discovery Today 2003, 8, 681) defined as "single molecular framework able to provide ligands for diverse receptors" (cf. B.E. Evans, K.E. Rittle, M.G. Bock, R.M. DiPardo, R.M. Freidinger, et al J. Med. Chem. 1988, 31, 2235). Therefore, indoles probably represent the most important of all structural classes in drug discovery (cf. A.L. Smith,G. I. Stevenson,C. J. Swain and J.L. Castro Tetrahedron Lett. 1998, 39, 8317).

Although a large number of drugs containig indole framework can be found within the field of oncology, such as vincristine (cf. H. Ishikawa et al. J. Am. Chem. Soc. 2009, 131, 4904), ellipticine (cf. M. G. Ferkin et al. ChemMedChem 2009, 4, 363; J. B. Le Pecq et al. Proc. Natl. Acad. Sci. USA 1974, 71, 5078*)* and many other compounds (see for example A. Ahmad, W. A. Sakr, K. M. Rahman Curr Drug Targets 2010, 11, 652; A.-R. Farghaly ARKIVOC 2010, 11, 177-187*;* Y.K. Chiang et al. J Med Chem 2009, 52, 4221; A. Stolle et al. (2002) PCT No. WO 2002030895), the interaction between DNMTs and non covalent inhibitors based on 1*H*-indole scaffolds and polyalkoxy and/or polyhydroxyphenyl groups is unknown.

The most widely explored DNMT1 inhibitors are azacitidine (Vidaza®) and decitabine (Dacogen®), nucleoside analogs that incorporate into DNA and irreversibly trap the enzyme due to formation of a covalent bond (cf. G. Egger et al. op. cit.*;* L. Zhou et al. J. Mol. Biol. 2002, 321, 591; R. Jüttermann et al Proc Natl Acad Sci USA 1994, 91, 11797). Both compounds are now used clinically for the treatment of myelodysplasic syndromes and lymphoproliferative diseases, but do possess considerable toxicity (cf. G. Leone et al. Clin. Immunol. 2003, 109, 89). Also, this kind of agents exerts poor activity on solid tumors, for example, in gastrointestinal malignancies (cf. X.P. Zou, B. Zhang, Y. Liu Chin. Med. J. 2010, 123, 1206*;* S.B. Baylin Nat. Clin. Pract. Oncol. 2005, 2 (suppl 1), s4).

Several other small-molecule inhibitors of DNA methylation have been described, whose general structures can be found in different reviews (cf. T. E. Fandy Curr. Med. Chem. 2009, 16, 2075; N. Yu, M. Wang Curr. Med. Chem. 2008, 15, 1350), including the psammaplin sponge metabolites (cf. Piña et al. J. Org. Chem. 2003, 68, 3866), which are potent direct inhibitors of DNA methyltransferases but are less effective in cellular assays (cf. A.M. Godert et al. Bioorg. Med. Chem. Lett. 2006, 16, 3330). Other non-nucleoside demethylating agents such as (-)-epigallocatechin-3-gallate (EGCG), hydralazine and procainamide were also shown to be far less effective in reactivating genes than decitabine (cf. J. C. Chuang et al Mol Cancer Ther 2005, 4, 1515). Non covalent small molecule inhibitors of DNMT are rarely found in the literature, and the ones that are reported sometimes suffer from weak potency and/or lack of selectivity. In addition, no structure-activity relationship can be envisaged, since the number of crystal structures of human DNMT and non covalent inhibitors is very scarce.

Thus, there still exists a need to develop effective non covalent DNMT inhibitors which can be used in the prevention or treatment of diseases associated with aberrant DNA methylation such as cancer, hematological malignancy, proliferative diseases, genetic diseases, neurological disorders and immunological disorders.

### OBJECT OF THE INVENTION

A first aspect of the invention refers to compounds of general formula (I), 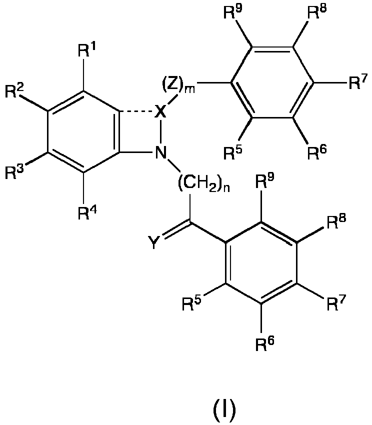 wherein:
X is a -(CH₂)ₙ- group or a -CH=CH- group;
Z is a C=Y group;
Y is a O atom or a N-OH group;
n=1-6
m is 0 or 1;
R¹-R⁹ represent, independently of each other, hydrogen, halogen, hydroxyl, alkoxyl or -OC(O)-alkyl,
with the proviso that:
when X is a -(CH₂)ₙ- group, m=1 and the dashed line does not represent a bond; and
when X is a -CH=CH- group, m=0 and the dashed line represents a carbon-carbon single bond, forming together with the benzyl group an indole ring;
or a solvate or a salt or prodrug thereof for its use as a medicament.

Another aspect of the invention refers to a compound of formula (I) as defined above for its use in the treatment of cancer, hematological malignancy, proliferative diseases, genetic diseases, neurological disorders and immunological disorders.

Another aspect of the present invention relates to the use of a compound of general formula (I) as defined above, or a salt, solvate or prodrug thereof, in the preparation of a medicament for the treatment of cancer, hematological malignancy and proliferative diseases, proliferative diseases, genetic diseases, neurological disorders and immunological disorders.

According to another aspect, the present invention is directed to a method of treating cancer, hematological malignancy proliferative diseases, genetic diseases, neurological disorders and immunological disorders, which comprises the administration to a patient needing such treatment, of a therapeutically effective amount of at least one compound of general formula (I) as defined above or a salt, solvate or prodrug thereof.

Another aspect of the invention refers to a compound of formula (I) as defined above or a solvate or a salt or prodrug thereof;

the following compounds being excluded when X is a -CH=CH- group:
R¹-R⁹=H Y=O and n=1;
R²=R⁴-R⁹=H, R¹=R³=OMe, Y=O and n=1;
R²=R⁴=R⁵=R⁶=R⁸=R⁹=H; R¹=R³=OMe R⁷=Br Y=O and n=1;
R²=R⁴=R⁵=R⁶=R⁸=R⁹=H, R¹=R³=OMe R⁷=Cl, Y=O and n=1;
and the following compounds being excluded when X is a -(CH₂)ₙ- group:
R¹-R⁹=H, Y=N-OH and n=1;
R¹-R⁹=H, Y=O and n=1;
R²=OMe; R¹ R³-R⁹=H, Y=N-OH and n=1;
R²=R⁷=OMe. R¹, R³-R⁶, R⁸-R⁹=H, Y=N-OH and n=1;
R²=OMe; R⁷=Cl; R¹, R³-R⁶, R⁸-R⁹=H, Y=N-OH and n=1;
R⁷=OMe; R¹-R⁶, R⁸-R⁹=H Y=O and n=1;
R⁷=Cl; R¹-R⁶, R⁸-R⁹=H, Y=O and n=1;
R¹=R³=OMe, R²=R⁴-R⁹=H, Y=O and n=1
R¹=R³=OMe R⁷=Br, R²=R⁴=R⁵=R⁶=R⁸=R⁹=H Y=O and n=1;
R²=OMe; R¹, R³-R⁹=H, Y=O and n=1;
R⁴=OH; R¹-R³, R⁵-R⁹=H, Y=O and n=1;
R²=R⁷=OH, R¹, R³-R⁶, R⁸-R⁹=H, Y=O and n=1.
R¹-R⁹=H, Y=O and n=2;
R²=OMe; R¹ R³-R⁹=H, Y=O and n=2;
R²=OMe, R⁷=Cl R¹, R³-R⁶, R⁸-R⁹=H, Y=O and n=2;
R²=OMe, R⁷=Br, R¹, R³-R⁶, R⁸-R⁹=H Y=O and n=2;
R²=R⁷=OMe, R¹, R³-R⁶, R⁸-R⁹=H, Y=O and n=2;
R²=Cl; R¹ R³-R⁹=H, Y=O and n=2;
R²=R⁷=Cl R¹, R³-R⁶, R⁸-R⁹=H, Y=O and n=2;
R²=Cl, R⁷=OMe, R¹, R³-R⁶, R⁸-R⁹=H, Y=O and n=2;
R²=Cl, R⁷=Br, R¹, R³-R⁶, R⁸-R⁹=H, Y=O and n=2;
R²=Br; R¹ R³-R⁹=H, Y=O and n=2;
R²=Br, R⁷=Cl R¹, R³-R⁶, R⁸-R⁹=H, Y=O and n=2;
R²=Br, R⁷=OMe, R¹, R³-R⁶, R⁸-R⁹=H, Y=O and n=2;
R²=R⁷=Br, R¹, R³-R⁶, R⁸-R⁹=H, Y=O and n=2.

Likewise, another aspect of the invention refers to the process for the preparation of compounds of general formula (I), or a solvate or a salt or prodrug thereof as those defined above.

A further object of the invention is a pharmaceutical composition comprising at least one compound of general formula (I) as those defined above, or a salt, solvate or prodrug thereof, and at least one pharmaceutically acceptable excipient.

### DETAILED DESCRIPTION OF THE INVENTION

First, the present invention provides compounds of general formula (I), 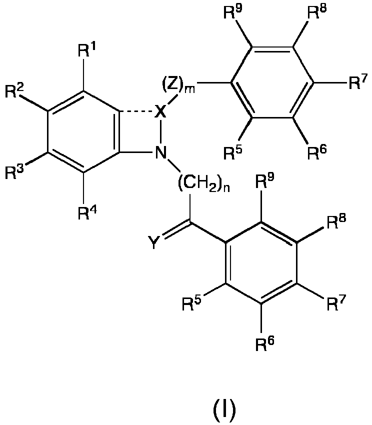 wherein:
X is a -(CH₂)ₙ- group or a -CH=CH- group;
Z is a C=Y group;
Y is a O atom or a N-OH group;
n=1-6
m is 0 or 1 ;
R¹-R⁹ represent, independently of each other, hydrogen, halogen, hydroxyl, alkoxyl or -OC(O)-alkyl,
with the proviso that:
when X is a -(CH₂)ₙ- group, m=1 and the dashed line does not represent a bond; and
when X is a -CH=CH- group, m=0 and the dashed line represents a carbon-carbon single bond, forming together with the benzyl group an indole ring;
or a solvate or a salt or prodrug thereof, for its use as a medicament.

The term "halogen" refers to -F, -Cl, -Br or -I.

The term "alkyl" refers to a linear or branched hydrocarbon chain radical consisting of carbon and hydrogen atoms, containing no unsaturation, having 1 to 6 carbon atoms, which is attached to the rest of the molecule by a single bond. Exemplary alkyl groups can be methyl, ethyl, n-propyl, or i-propyl,

The term "alkoxyl" refers to a radical of the formula -O-alkyl, wherein "alkyl" is as defined above. In an embodiment of the invention, alkoxyl refers to a radical of formula -O-C₁-C₃ alkyl. Exemplary alkoxyl radicals are methoxyl, ethoxyl, n-propoxyl or i-propoxyl.

According to a particular embodiment, at least one of R¹, R², R³ and R⁴ is not hydrogen.

In another particular embodiment, at least one of R⁵, R⁶, R⁷, R⁸ or R⁹ is not hydrogen.

In another particular embodiment, at least one of R¹, R², R³ and R⁴ is selected from halogen, preferably fluor, hydroxyl and alkoxyl.

According to a further embodiment, at least one of R⁵, R⁶, R⁷, R⁸ and R⁹ is selected from halogen, preferably fluor, hydroxyl, alcoxyl and -OC(O)-alkyl, more preferably at least one of R⁵, R⁶, R⁷, R⁸ and R⁹ is selected from halogen, preferably fluor, hydroxyl and -OC(O)-alkyl.

According to a particular embodiment, at least one of R¹, R², R³ and R⁴ is an alkoxyl group, and at least one of R⁵, R⁶, R⁷, R⁸ and R⁹ is a hydroxyl group. According to a particular embodiment, R³ is an alkoxyl group, and at least one of R⁵, R⁶, R⁷, R⁸ and R⁹ is a hydroxyl group. According to a particular embodiment, R¹ and R³ are alkoxyl groups, and at least one of R⁵, R⁶, R⁷, R⁸ and R⁹ is a hydroxyl group.

According to a particular embodiment, at least one of R¹, R², R³ and R⁴ is an alkoxyl group, and at least one of R⁵, R⁶, R⁷, R⁸ and R⁹ is -OC(O)-alkyl. According to a particular embodiment, R³ is an alkoxyl group, and at least one of R⁵, R⁶, R⁷, R⁸ and R⁹ is -OC(O)-alkyl. According to a particular embodiment, R¹ and R³ are alkoxyl groups, and at least one of R⁵, R⁶, R⁷, R⁸ and R⁹ is - OC(O)-alkyl.

According to another particular embodiment, each alkoxyl group is independently -O-C₁-C₃ alkyl, preferably methoxyl.

In a preferred embodiment, the compounds of general formula (I) are selected from:
[1] 1-(4-Hydroxyphenyl)-2-[3-(4-hydroxyphenyl)-6-methoxy-1*H*-indol-1-yl] ethanone, with the following structural formula: 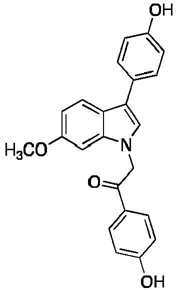
[2] 1-(3-Hydroxyphenyl)-2-[3-(3-hydroxyphenyl)-6-methoxy-1*H*-indol-1-yl] ethanone, with the following structural formula: 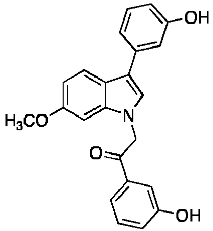
[3] 1-(2-Hydroxyphenyl)-2-[3-(2-hydroxyphenyl)-6-methoxy-1*H*-indol-1-yl] ethanone, with the following structural formula: 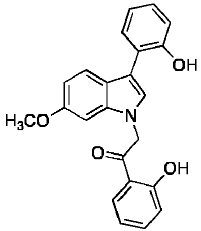
[4] 1-(3,4-Dihydroxyphenyl)-2-[3-(3,4-dihydroxyphenyl)-6-methoxy-1*H*-indol-1-yl]ethanone, with the following structural formula: 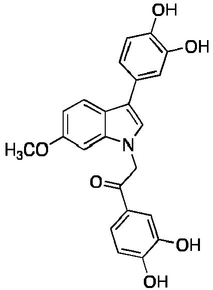
[5] 2-[6-Methoxy-3-(2,3,4-trihydroxyphenyl)-1*H*-indol-1-yl]-1-(2,3,4-trihydroxyphenyl)ethanone, with the following structural formula: 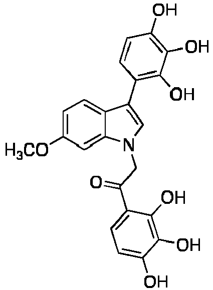
[6] 1-(4-Hydroxyphenyl)-2-[3-(4-hydroxyphenyl)-4,6-dimethoxy-1*H*-indol-1-yl]ethanone, with the following structural formula: 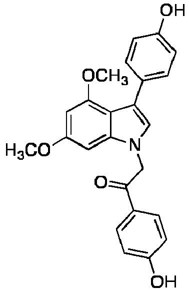
[7] 1-(2-Hydroxyphenyl)-2-[3-(2-hydroxyphenyl)-4,6-dimethoxy-1*H*-indol-1-yl]ethanone, with the following structural formula: 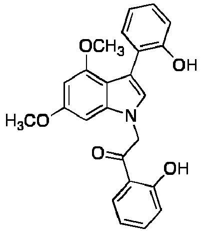
[8] 1-(3,4-Dihydroxyphenyl)-2-[3-(3,4-dihydroxyphenyl)-4,6-dimethoxy-1H-indol-1-yl]ethanone, with the following structural formula: 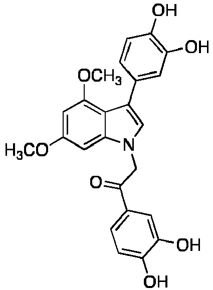
[9] 1-(2,4-Dihydroxyphenyl)-2-[3-(2,4-dihydroxyphenyl)-4,6-dimethoxy-1H-indol-1-yl]ethanone, with the following structural formula: 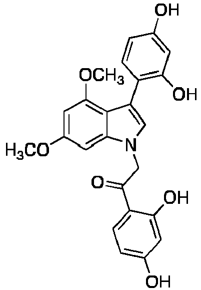
[10] 1-(3,5-dihydroxyphenyl)-2-(3-(3,5-dihydroxyphenyl)-4,6-dimethoxy-1H-indol-1-yl)ethanone, with the following structural formula: 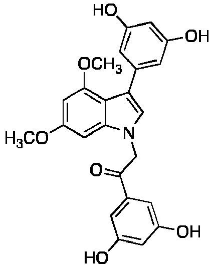
[11] 2-[4,6-Dimethoxy-3-(2,3,4-trihydroxyphenyl)-1*H*-indol-1-yl]-1-(2,3,4-trihydroxyphenyl)ethanone, with the following structural formula: 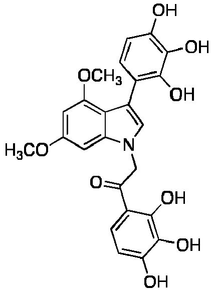
[12] 1-(4-Fluorophenyl)-2-[3-(4-fluorophenyl)-4,6-dimethoxy-1*H*-indol-1-yl] ethanone, with the following structural formula: 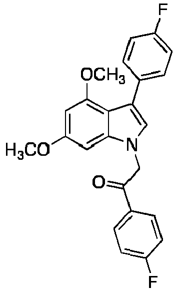
[13] 1-(4-Hydroxyphenyl)-2-[3-(4-hydroxyphenyl)-4,7-dimethoxy-1H-indol-1-yl]ethanone, with the following structural formula: 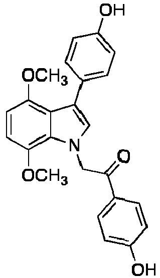
[14] 1-(4-Hydroxyphenyl)-2-[3-(4-hydroxyphenyl)-5,6-dimethoxy-1*H*-indol-1-yl]ethanone, with the following structural formula: 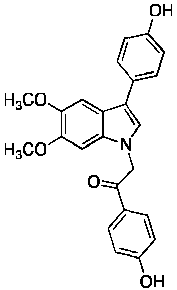
[15] 2-[5-Hydroxy-3-(4-hydroxyphenyl)-6-methoxy-1*H*-indol-1-yl]-1-(4-hydroxyphenyl)ethanone, with the following structural formula: 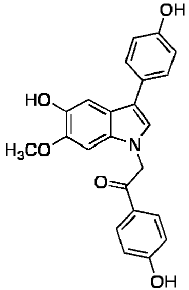
[16] 2-[4,6-Difluoro-3-(4-hydroxyphenyl)-1*H*-indol-1-yl]-1-(4-hydroxyphenyl) ethanone, with the following structural formula: 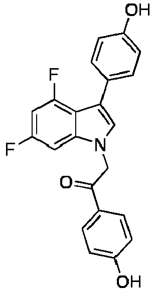
[17] 2-[6-Hydroxy-3-(4-hydroxyphenyl)-1*H*-indol-1-yl]-1-(4-hydroxyphenyl) ethanone, with the following structural formula: 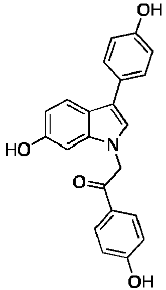
[18] 2-[5-Hydroxy-3-(4-hydroxyphenyl)-1*H*-indol-1-yl]-1-(4-hydroxyphenyl) ethanone, with the following structural formula: 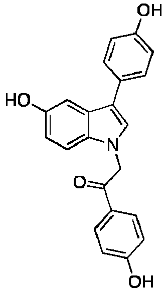
[19] 2-[5-Fluoro-3-(4-hydroxyphenyl)-1*H*-indol-1-yl]-1-(4-hydroxyphenyl) ethanone, with the following structural formula: 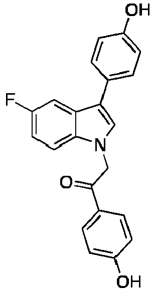
[20] 1-(4-Hydroxyphenyl)-2-[3-(4-hydroxyphenyl)-1*H*-indol-1-yl]ethanone, with the following structural formula: 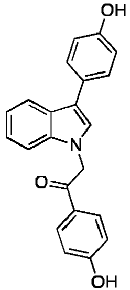
[21] ((*E*)-1-(4-Hydroxyphenyl)-2-[3-(4-hydroxyphenyl)-4,6-dimethoxy-1H-indol-1-yl]ethanone oxime, with the following structural formula: 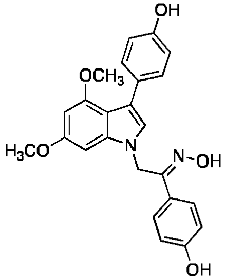
[22] ((Z)-1-(4-Hydroxyphenyl)-2-[3-(4-hydroxyphenyl)-4,6-dimethoxy-1H-indol-1-yl]ethanone oxime, with the following structural formula: 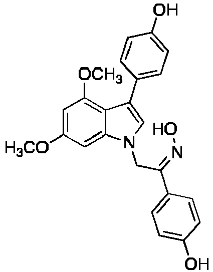
[23] 2,2'-[(3-Methoxyphenyl)azanediyl]-bis[1-(2,3,4-trihydroxyphenyl) ethanone], with the following structural formula: 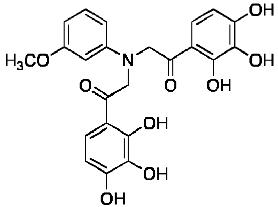
[24] 2,2'-[(3,5-dimethoxyphenyl)azanediyl]-bis[1-(2,3,4-trihydroxyphenyl) ethanone], with the following structural formula: 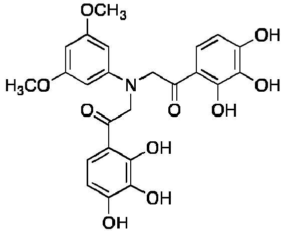
[25] Potassium salt of 1-(4-hydroxyphenyl)-2-[3-(4-hydroxyphenyl)-6-methoxy-1*H*-indol-1-yl] ethanone
[26] Potassium salt of 1-(4-hydroxyphenyl)-2-[3-(4-hydroxyphenyl)-4,6-dimethoxy-1*H*-indol-1-yl] ethanone
[27] Calcium salt of 1-(4-hydroxyphenyl)-2-[3-(4-hydroxyphenyl)-4,6-dimethoxy-1*H*-indol-1-yl] ethanone
[28] 4-{1-[2-(4-Acetoxyphenyl)-2-oxoethyl]-6-methoxy-1*H*-indol-3-yl}phenyl acetate, with the following structural formula: 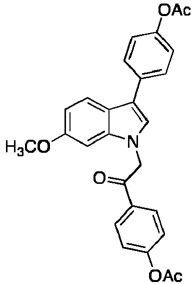
[29] 4-{1-[2-(4-Acetoxyphenyl)-2-oxoethyl]-4,6-dimethoxy-1*H*-indol-3-yl} phenyl acetate, with the following structural formula: 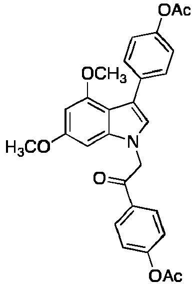
[30] 4-{1-[2-(3,4-Diacetoxyphenyl)-2-oxoethyl]-4,6-dimethoxy-1*H*-indol-3-yl}-1,2-phenylene diacetate, with the following structural formula: 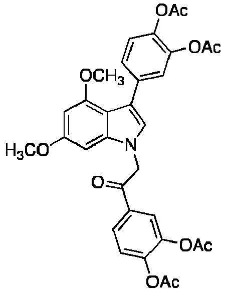
[31] 4-{1-[2-(3,4-Diacetoxyphenyl)-2-oxoethyl]-6-methoxy-1*H*-indol-3-yl}-1,2-phenylene diacetate, with the following structural formula: 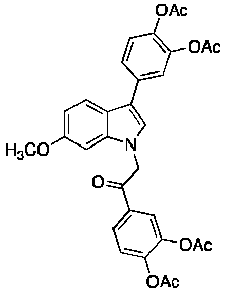
or a solvate or a salt or prodrug thereof.

The compounds of formula (I) defined above may be in the form of solvates or salts or prodrugs, preferably as a pharmaceutically acceptable species.

The term "pharmaceutically acceptable species" refers to compositions and molecular entities that are physiologically tolerable and do not typically produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The term "prodrug" is used in its broadest sense and encompasses those derivatives that are converted *in vivo* into the compounds of the invention. Experts in the art would readily produce such derivatives, and include, depending on the functional groups present in the molecule and without limitation, the following derivatives of the present compounds: disulfides, thioesters, esters, amino acid esters, phosphate esters, esters of metallic salt sulfonates, carbamates and amides.

The term "solvate" means any form of the active compound of the invention which has another molecule (for example a polar solvent such as water or ethanol, a cyclodextrin or a dendrimer) attached to it through noncovalent bonds. Methods of solvation are known within the art.

Compounds of formula (I) may also be in the form of salts. Non-limiting examples are sulphates; hydrohalide salts; phosphates; lower alkane sulphonates; arylsulphonates; salts of C₁-C₂₀ aliphatic mono-, di- or tribasic acids which may contain one or more double bonds, an aryl nucleus or other functional groups such as hydroxy, amino, or keto; salts of aromatic acids in which the aromatic nuclei may or may not be substituted with groups such as hydroxyl, lower alkoxyl, amino, mono- or di- lower alkylamino sulphonamido. Also included within the scope of the invention are quaternary salts of the tertiary nitrogen atom with lower alkyl halides or sulphates, and oxygenated derivatives of the tertiary nitrogen atom, such as the N-oxides. In preparing dosage formulations, those skilled in the art will select the pharmaceutically acceptable salts.

Solvates, salts and prodrugs can be prepared by methods known in the state of the art. Note that the non-pharmaceutically acceptable solvates and prodrugs also fall within the scope of the invention because they can be useful in preparing pharmaceutically acceptable salts, solvates or prodrugs.

The compounds of formula (I) defined above also seek to include compounds that differ only in the presence of one or more isotopically enriched atoms. For example, compounds having the present structures except for the replacement of a hydrogen by a deuterium or tritium, or the replacement of a carbon by a carbon enriched in ¹¹C, ¹³C or ¹⁴C or a ¹⁵N enriched nitrogen are within the scope of this invention.

Another aspect of the invention refers to the compounds of formula (I) as defined above for its use in a variety of therapeutic applications. According to a particular embodiment, the compounds of general formula (I) are useful for the treatment of various types of cancer, hematological malignancy, proliferative diseases, genetic diseases, neurological disorders and immunological disorders, by changing the methylation pattern of DNA regions involved in the mentioned diseases.

Methylation changes in gene promoter regions can modify the gene expression of many classic tumor-suppressor genes, androgen and estrogen receptor genes, cell adhesion genes, cell-cycle control genes or apoptotic genes. These modifications may restrict tumor growth and metastasis or may activate mechanisms of apoptosis induction or other processes that stop the development of primary or metastatic tumors.

According to a particular embodiment, the cancer is selected from breast cancer, chronic myelogenous (or myeloid) leukemia (CML), colorectal cancer, fibrosarcoma, gastric cancer, glioblastoma, kidney cancer, liver cancer, lung cancer, melanoma, nasopharyngeal cancer, oral cancer, orthotopic multiple myeloma, osteosarcoma, ovarian cancer, pancreatic cancer, and prostate cancer.

According to an embodiment of the invention, the neurological disorder is schizophrenia, fragile X syndrome or Alzheimer.

According to an embodiment of the invention, the genetic disease is ATR-X, Rett, Fragile X, Prader-Willi, Angelman, CHARGE, CSB, SIOD (Schimke Immuno-Osseous Dysplasia), ICF, Rubinstein-Taybi syndrome or FSHD (Facioscapulo-humeral Dystrophy).

According to an embodiment of the invention, the immunological disorder is lupus erythematosus (SLE), rheumatoid arthritis or progressive systemic sclerosis (PSS).

Another aspect of the present invention refers to a compound of formula (I): 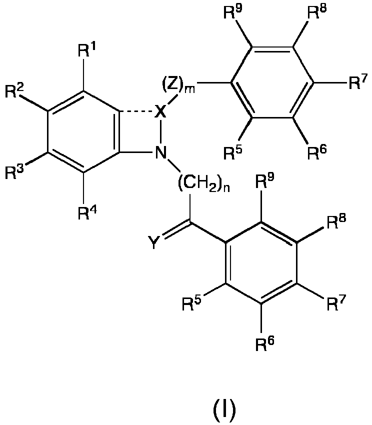 wherein:
X is a -(CH₂)ₙ- group or a -CH=CH- group;
Z is a C=Y group;
Y is a O atom or a N-OH group;
n=1-6
m is 0 or 1;
R¹-R⁹ represent, independently of each other, hydrogen, halogen, hydroxyl, alkoxyl or -OC(O)-alkyl,
with the proviso that:
when X is a -(CH₂)ₙ- group, m=1 and the dashed line does not represent a bond; and
when X is a -CH=CH- group, m=0 and the dashed line represents a carbon-carbon single bond, forming together with the benzyl group an indole ring;
or a solvate or a salt or prodrug thereof,
the following compounds being excluded when X is a -CH=CH- group:
R¹-R⁹=H, Y=O and n=1;
R²=R⁴-R⁹=H, R¹=R³=OMe, Y=O and n=1;
R²=R⁴=R⁵=R⁶=R⁸=R⁹=H; R¹=R³=OMe, R⁷=Br, Y=O and n=1;
R²=R⁴=R⁵=R⁶=R⁸=R⁹=H, R¹=R³=OMe R⁷=Cl, Y=O and n=1;
and the following compounds being excluded when X is a -(CH₂)ₙ- group:
R¹-R⁹=H, Y=N-OH and n=1;
R¹-R⁹=H Y=O and n=1;
R²=OMe; R¹, R³-R⁹=H, Y=N-OH and n=1;
R²=R⁷=OMe; R¹, R³-R⁶, R⁸-R⁹=H Y=N-OH and n=1;
R²=OMe; R⁷=Cl; R¹, R³-R⁶, R⁸-R⁹=H, Y=N-OH and n=1;
R⁷=OMe. R¹-R⁶, R⁸-R⁹=H, Y=O and n=1;
R⁷=Cl; R¹-R⁶, R⁸-R⁹=H, Y=O and n=1;
R¹=R³=OMe, R²=R⁴-R⁹=H, Y=O and n=1
R¹=R³=OMe, R⁷=Br, R²=R⁴=R⁵=R⁶=R⁸=R⁹=H Y=O and n=1;
R²=OMe; R¹, R³-R⁹=H, Y=O and n=1;
R⁴=OH; R¹-R³, R⁵-R⁹=H, Y=O and n=1;
R²=R⁷=OH, R¹, R³-R⁶, R⁸-R⁹=H, Y=O and n=1.
R¹-R⁹=H, Y=O and n=2;
R²=OMe; R¹, R³-R⁹=H, Y=O and n=2;
R²=OMe, R⁷=Cl, R¹, R³-R⁶ R⁸-R⁹=H, Y=O and n=2;
R²=OMe, R⁷=Br R¹, R³-R⁶, R⁸-R⁹=H, Y=O and n=2;
R²=R⁷=OMe, R¹, R³-R⁶, R⁸-R⁹=H, Y=O and n=2;
R²=Cl; R¹, R³-R⁹=H, Y=O and n=2;
R²=R⁷=Cl, R¹, R³-R⁶ R⁸-R⁹=H, Y=O and n=2;
R²=Cl, R⁷=OMe, R¹, R³-R⁶, R⁸-R⁹=H, Y=O and n=2;
R²=Cl, R⁷=Br R¹, R³-R⁶, R⁸-R⁹=H, Y=O and n=2;
R²=Br; R¹ R³-R⁹=H, Y=O and n=2;
R²=Br, R⁷=Cl R¹, R³-R⁶, R⁸-R⁹=H, Y=O and n=2;
R²=Br, R⁷=OMe, R¹, R³-R⁶, R⁸-R⁹=H, Y=O and n=2;
R²=R⁷=Br, R¹ R³-R⁶, R⁸-R⁹=H Y=O and n=2.

According to a particular embodiment, at least one of R¹, R² R³ and R⁴ is not hydrogen.

In another particular embodiment, at least one of R⁵, R⁶, R⁷, R⁸ and R⁹ is not hydrogen.

In another particular embodiment, at least one of R¹, R², R³ and R⁴ is selected from halogen, preferably fluor, hydroxyl and alkoxyl.

According to a further embodiment, at least one of R⁵, R⁶, R⁷, R⁸ and R⁹ is selected from halogen, preferably fluor, hydroxyl, alcoxyl and -OC(O)-alkyl, more preferably at least one of R⁵, R⁶, R⁷, R⁸ and R⁹ is selected from halogen, preferably fluor, hydroxyl and -OC(O)-alkyl.

According to a particular embodiment, at least one of R¹, R², R³ and R⁴ is an alkoxyl group, and at least one of R⁵, R⁶, R⁷, R⁸ and R⁹ is a hydroxyl group. According to a particular embodiment, R³ is an alkoxyl group, and at least one of R⁵, R⁶, R⁷, R⁸ and R⁹ is a hydroxyl group. According to a particular embodiment, R¹ and R³ are alkoxyl groups, and at least one of R⁵, R⁶, R⁷, R⁸ and R⁹ is a hydroxyl group.

According to a particular embodiment, at least one of R¹, R², R³ and R⁴ is an alkoxyl group, and at least one of R⁵, R⁶, R⁷, R⁸ and R⁹ is -OC(O)-alkyl. According to a particular embodiment, R³ is an alkoxyl group, and at least one of R⁵, R⁶, R⁷, R⁸ and R⁹ is -OC(O)-alkyl. According to a particular embodiment, R¹ and R³ are alkoxyl groups, and at least one of R⁵, R⁶, R⁷, R⁸ and R⁹ is - OC(O)-alkyl.

According to another particular embodiment, each alkoxyl group is independently -O-C₁-C₃ alkyl, preferably methoxy.

In a preferred embodiment, the compounds of general formula (I) of the invention are selected from:
[1] 1-(4-Hydroxyphenyl)-2-[3-(4-hydroxyphenyl)-6-methoxy-1*H*-indol-1-yl] ethanone, with the following structural formula:
[2] 1-(3-Hydroxyphenyl)-2-[3-(3-hydroxyphenyl)-6-methoxy-1*H*-indol-1-yl] ethanone, with the following structural formula:
[3] 1-(2-Hydroxyphenyl)-2-[3-(2-hydroxyphenyl)-6-methoxy-1*H*-indol-1-yl] ethanone, with the following structural formula:
[4] 1-(3,4-Dihydroxyphenyl)-2-[3-(3,4-dihydroxyphenyl)-6-methoxy-1*H*-indol-1-yl]ethanone, with the following structural formula:
[5] 2-[6-Methoxy-3-(2,3,4-trihydroxyphenyl)-1H-indol-1-yl]-1-(2,3,4-trihydroxyphenyl)ethanone, with the following structural formula:
[6] 1-(4-Hydroxyphenyl)-2-[3-(4-hydroxyphenyl)-4,6-dimethoxy-1*H*-indol-1-yl]ethanone, with the following structural formula:
[7] 1-(2-Hydroxyphenyl)-2-[3-(2-hydroxyphenyl)-4,6-dimethoxy-1*H*-indol-1-yl]ethanone, with the following structural formula:
[8] 1-(3,4-Dihydroxyphenyl)-2-[3-(3,4-dihydroxyphenyl)-4,6-dimethoxy-1H-indol-1-yl]ethanone, with the following structural formula:
[9] 1-(2,4-Dihydroxyphenyl)-2-[3-(2,4-dihydroxyphenyl)-4,6-dimethoxy-1H-indol-1-yl]ethanone, with the following structural formula:
[10] 1-(3,5-dihydroxyphenyl)-2-(3-(3,5-dihydroxyphenyl)-4,6-dimethoxy-1H-indol-1-yl)ethanone, with the following structural formula:
[11] 2-[4,6-Dimethoxy-3-(2,3,4-trihydroxyphenyl)-1*H*-indol-1-yl]-1-(2,3,4-trihydroxyphenyl)ethanone, with the following structural formula:
[12] 1-(4-Fluorophenyl)-2-[3-(4-fluorophenyl)-4,6-dimethoxy-1*H*-indol-1-yl] ethanone, with the following structural formula:
[13] 1-(4-Hydroxyphenyl)-2-[3-(4-hydroxyphenyl)-4,7-dimethoxy-1*H*-indol-1-yl]ethanone, with the following structural formula:
[14] 1-(4-Hydroxyphenyl)-2-[3-(4-hydroxyphenyl)-5,6-dimethoxy-1H-indol-1-yl]ethanone, with the following structural formula:
[15] 2-[5-Hydroxy-3-(4-hydroxyphenyl)-6-methoxy-1*H*-indol-1-yl]-1-(4-hydroxyphenyl)ethanone, with the following structural formula:
[16] 2-[4,6-Difluoro-3-(4-hydroxyphenyl)-1*H-*indol-1-yl]-1-(4-hydroxyphenyl) ethanone, with the following structural formula:
[17] 2-[6-Hydroxy-3-(4-hydroxyphenyl)-1*H*-indol-1-yl]-1-(4-hydroxyphenyl) ethanone, with the following structural formula:
[18] 2-[5-Hydroxy-3-(4-hydroxyphenyl)-1*H*-indol-1-yl]-1-(4-hydroxyphenyl) ethanone, with the following structural formula:
[19] 2-[5-Fluoro-3-(4-hydroxyphenyl)-1*H*-indol-1-yl]-1-(4-hydroxyphenyl) ethanone, with the following structural formula:
[20] 1-(4-Hydroxyphenyl)-2-[3-(4-hydroxyphenyl)-1*H*-indol-1-yl]ethanone, with the following structural formula:
[21] ((*E*)-1-(4-Hydroxyphenyl)-2-[3-(4-hydroxyphenyl)-4,6-dimethoxy-1H-indol-1-yl]ethanone oxime, with the following structural formula:
[22] ((Z)-1-(4-Hydroxyphenyl)-2-[3-(4-hydroxyphenyl)-4,6-dimethoxy-1H-indol-1-yl]ethanone oxime, with the following structural formula:
[23] 2,2'-[(3-Methoxyphenyl)azanediyl]-bis[1-(2,3,4-trihydroxyphenyl) ethanone], with the following structural formula:
[24] 2,2'-[(3,5-dimethoxyphenyl)azanediyl]-bis[1-(2,3,4-trihydroxyphenyl) ethanone], with the following structural formula:
[25] Potassium salt of 1-(4-hydroxyphenyl)-2-[3-(4-hydroxyphenyl)-6-methoxy-1*H*-indol-1-yl] ethanone
[26] Potassium salt of 1-(4-hydroxyphenyl)-2-[3-(4-hydroxyphenyl)-4,6-dimethoxy-1*H*-indol-1-yl] ethanone
[27] Calcium salt of 1-(4-hydroxyphenyl)-2-[3-(4-hydroxyphenyl)-4,6-dimethoxy-1*H*-indol-1-yl] ethanone
[28] 4-{1-[2-(4-Acetoxyphenyl)-2-oxoethyl]-6-methoxy-1*H-*indol-3-yl}phenyl acetate, with the following structural formula:
[29] 4-{1-[2-(4-Acetoxyphenyl)-2-oxoethyl]-4,6-dimethoxy-1*H*-indol-3-yl} phenyl acetate, with the following structural formula:
[30] 4-{1-[2-(3,4-Diacetoxyphenyl)-2-oxoethyl]-4,6-dimethoxy-1*H*-indol-3-yl}-1,2-phenylene diacetate, with the following structural formula:
[31] 4-{1-[2-(3,4-Diacetoxyphenyl)-2-oxoethyl]-6-methoxy-1*H*-indol-3-yl}-1,2-phenylene diacetate, with the following structural formula:
or a solvate or a salt or prodrug thereof.

### Synthesis of compounds of formula (I)

Another aspect of the invention refers to procedures to obtain compounds of general formula (I) of the invention. The following methods A, B, C and D describe the procedures for obtaining compounds of general formula (I), among which include compounds of formula (Ia), (Ib), (Ic) and I(d), or solvates or salts or prodrugs thereof.

Compounds of formula (Ia) correspond to compounds of formula (I), wherein R¹-R⁹ and n have the meaning given above, X is -CH=CH-, Y=O and m=0.

Compounds of formula (Ib) correspond to compounds of formula (I), wherein R¹-R⁹ and n have the meaning given above, X is -(CH₂)ₙ, Y=O and m=1.

Compounds of formula (Ic) correspond to compounds of formula (I), wherein R¹-R⁹ and n have the meaning given above, X is -CH=CH-, Y=-N-OH and m=0.

Compounds of formula (Id) correspond to compounds of formula (I), wherein R¹-R⁹ and n have the meaning given above, X is -(CH₂)ₙ, Y=-N-OH and m=1.

### Method A

Method A represents a procedure for the preparation of compounds of general formula (Ia): 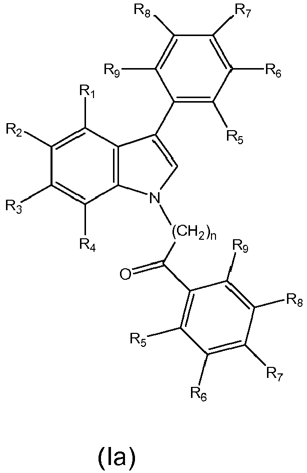 wherein R¹-R⁹ and n have the meaning given above, which comprises reacting:
a) a compound of general formula (II), 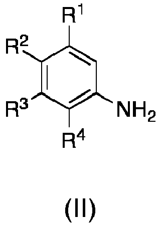 wherein R¹, R², R³ and R⁴ have the meaning given above;
   with
b) a compound of general formula (III), 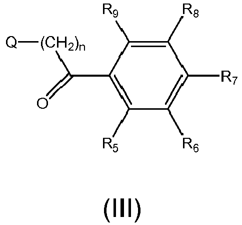 wherein Q is a chlorine, bromine or iodine atom, or a leaving group, such as mesylate or tosylate, and R⁵, R⁶, R⁷, R⁸, R⁹ have the meaning given above;
   in the presence of
c) a base, either organic or inorganic; and
d) an appropriate solvent
leaving the reaction to react for at least 16 hours.

### Method B

Method B represents a procedure for the preparation of compounds of general formula (Ib): 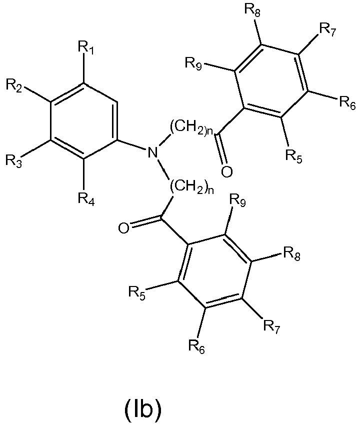 wherein R¹-R⁹ and n have the meaning given above, which comprises reacting:
a) a compound of general formula (II), 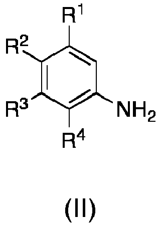 wherein R¹, R², R³ and R⁴ have the meaning given above;
   with
b) a compound of general formula (III), 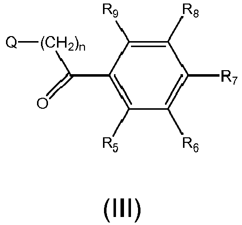 wherein Q is a chlorine, bromine or iodine atom, or a leaving group, such as mesylate or tosylate, and R⁵, R⁶, R⁷, R⁸, R⁹ have the meaning given above;
   in the presence of
c) a base, either organic or inorganic; and
d) an appropriate solvent.
leaving the reaction to react for three hours at the most.

The reaction defined in Method A and Method B usually takes place at temperatures ranging from 25°C to +170°C until completion of the reaction.

For the aims of the invention, the reaction mixture defined in Method A and Method B made up of the four compounds of phases a) to d) can be made by adding one of the components to the mixture formed by the three other components at a temperature ranging from +25°C to +170°C. After completion of the addition, the resulting mixture is stirred until completion of the reaction.

The base used in Methods A and B can be selected among inorganic or organic bases. The inorganic base may be selected from the group consisting of carbonates of alkaline metals or alkaline earth metals (e.g. sodium, lithium, potassium, calcium, or magnesium carbonate), bicarbonates of alkaline metals (e.g. sodium, lithium or potassium bicarbonate), sulfates of alkaline metals or alkaline earth metals (e.g. sodium, lithium, potassium, calcium, or magnesium sulfate), acetates of alkaline metals or alkaline earth metals (e.g. sodium, lithium, potassium, calcium, or magnesium acetate), hydroxides of alkaline metals or alkaline earth metals (e.g. sodium, lithium, potassium, calcium, or magnesium hydroxide) or phosphates, monohydrogen phosphates or dihydrogen phosphates of alkaline metals or alkaline earth metals (e.g. sodium, lithium, potassium, calcium, or magnesium phosphate, or potassium dihydrogen phosphate). The organic base may be a primary, secondary or tertiary amine, preferably a tertiary amine selected from among the cyclic or acyclic aliphatic amines with C₃-C₁₀ carbon atoms and the alkanoaromatic amines with C₉-C₁₅ carbon atoms, more preferably N,N-dimethylaniline, triethylamine, *N,N-*diisopropyl ethylamine (DIPEA), N-methyl morpholine, N-methylpyrrolidine, 1,8-diazabicyclo[5.4.0] undec-7-ene (DBU), pyridine

The solvent used in Methods A and B can be a polar protic solvent such an alcohol, for example ethanol or other liquid alcohol at room temperature, a polar nonprotic solvent such a cyclic or acyclic ether, N,N-dimethylformamide or 1,2-dimethoxyethane, or a nonpolar solvent such as a linear or branched aliphatic hydrocarbon of C₅-C₁₀ carbons or an aromatic hydrocarbon such as toluene, xylene or similar.

### Method C

Method C represents a procedure for the preparation of compounds of general formula (Ic): 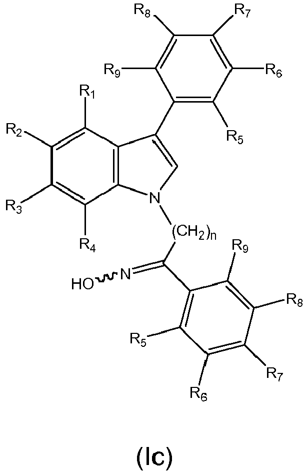 wherein R¹-R⁹ have the meaning given in the description of Method A, which comprises:
a) preparing a compound of general formula (Ia) as described in Method A; and
b) reacting the mentioned compound of formula (Ia) with a mixture of hydroxylamine hydrochloride and phenolphthalein in the presence of an excess of sodium methoxide in methanol.

### Method D

Method D represents a procedure for the preparation of compounds of general formula (Id): 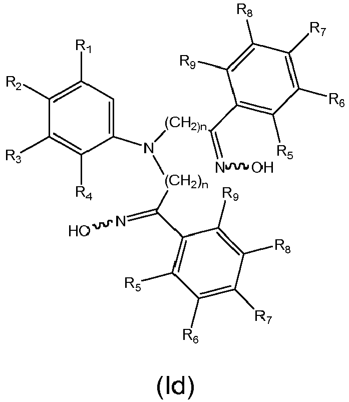 wherein R¹-R⁹ have the meaning given in the description of Method B, which comprises:
a) preparing a compound of general formula (Ib) as described in Method B; and
b) reacting the mentioned compound of formula (Ib) with a mixture of hydroxylamine hydrochloride and phenolphthalein in the presence of an excess of sodium methoxide in methanol.

For the aims of the invention, and regarding Methods C and D, the ketone of general formula (Ia) or (Ib) is added to a mixture of hydroxylamine hydrochloride and phenolphthalein in the presence of an excess of sodium methoxide in methanol. Upon completion of the reaction, after the corresponding treatment, compounds of formula (Ic) and (Id) are obtained.

In a particular embodiment, when any of R⁵-R⁹ is -OC(O)-alkyl, said radical can be obtained from the hydroxyl group. In particular, hydroxyl groups can be protected by known procedures, for example, by treatment with the corresponding anhydride. In an embodiment of the invention the reaction takes place using acetic anhydride and a aromatic organic base, preferably pyridine (see for example T.G. Bonner and P. McNamara J. Chem. Soc. B 1968, 7, 795; H. Chung and N.R. Washburn ACS Appl. Mater. Interfaces 2012, 4, 2840) at a temperature ranging from 0°C to +40°C.

A further embodiment of the invention is a salt of a compound of formula (I) of the invention. According to a particular embodiment, the salt is a phenoxy salt of alkaline metals or alkaline earth metals. To obtain the salts corresponding to compounds of formula (I) wherein at least one of the R¹-R⁹ is a hydroxyl group, hydroxyl groups can be treated with hydroxides of alkaline metals or alkaline earth metals (e.g. sodium, lithium, potassium, calcium, or magnesium hydroxide) at a temperature ranging from 0°C to +40°C. In an embodiment of the invention the reaction takes place at room temperature using water as solvent.

The initial compounds and starting materials, e.g. the compounds of formula (II) and (III), are either commercially available or can be obtained following procedures described in the literature. For example, see Chen L., Ding Q., Gillespie P., Kim K., Lovey A. J., McComas W. W., Mullin J. G. and Perrota A., (2002) PCT No. WO 2002057261 (e.g. Examples 7-13, pages 46-50; or Examples 14H-140, pages 57-60); King L. C., Ostrum G. K. J. Org. Chem. 1964, 29, 3459-3461; Diwu Z., Beachdel C., Klaubert D.H. Tetrahedron Lett. 1998, 39, 4987-4990; Bakke B. A., McIntosh M. C., Turnbull K.D. J. Org. Chem. 2005, 70(1), 4338-4345).

### Pharmaceutical compositions

Another aspect of the present invention refers to a pharmaceutical composition which comprises the compounds of formula (I) of the invention, or a pharmaceutically acceptable solvate or salt or prodrug thereof, and at least a pharmaceutically acceptable excipient.

The term "excipient" refers to a vehicle, diluent or adjuvant that is administered with the active ingredient. Such pharmaceutical excipients can be sterile liquids, such as water and oils, including those of petroleum, animal, vegetable or synthetic origin, such as peanut oil, soybean oil, mineral oil, sesame oil and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005; or "Handbook of Pharmaceutical Excipients", Rowe C. R.; Paul J. S.; Marian E. Q., sixth Edition.

Examples of pharmaceutical compositions include any solid composition (tablets, pills, capsules, granules, etc.) or liquid composition (solutions, suspensions or emulsions) for oral, topical or parenteral administration.

In a preferred embodiment the pharmaceutical compositions are in oral delivery form. Pharmaceutical forms suitable for oral administration may be tablets and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate.

Solid oral compositions can be prepared by conventional methods of blending, filling or preparation of tablets. Repeated blending operations can be used to distribute the active ingredient in all the compositions that use large amounts of fillers. Such operations are conventional in the art. The tablets can be prepared, for example, by dry or wet granulation and optionally can be coated by well known methods in normal pharmaceutical practice, in particular using a enteric coating.

Pharmaceutical compositions can also be adapted for parenteral administration, such as sterile solutions, suspensions or lyophilized products in the appropriate unit dosage form. Suitable excipients such as fillers, buffering agents or surfactants can be used.

The mentioned formulations will be prepared using standard methods such as those described or referred to in the Spanish and U.S. Pharmacopoeias and similar reference texts.

In general, the effective amount of a compound of the invention to be administered will depend on the relative efficacy of the compound chosen, the severity of the disorder being treated and the patient's weight. However, the active compounds will normally be administered one or more times a day, for example 1, 2, 3 or 4 times daily, with typical total daily doses in the range from 0.01 up to 1000 mg/kg/day.

The compounds of the present invention can be used with at least another drug to provide a combination therapy. This other drug or drugs may be part of the same pharmaceutical composition, or may be provided as a separate composition and can be administered at the same time or at different times.

The term "treatment" or "treating" in the context of this document means administration of a compound or a formulation according to this invention to prevent, improve or eliminate the disease or one or more symptoms associated with the disease. "Treatment" also encompasses preventing, improving or eliminating the physiological sequelae of the disease.

In order to facilitate the understanding of the preceding ideas, some examples of experimental procedures and embodiments of the present invention are described below. These examples are merely illustrative.

### EXAMPLES

### General Synthesis Methods

### Method A: Synthesis of indoles

### A.1) Synthesis of 1,3-disubstituted indoles

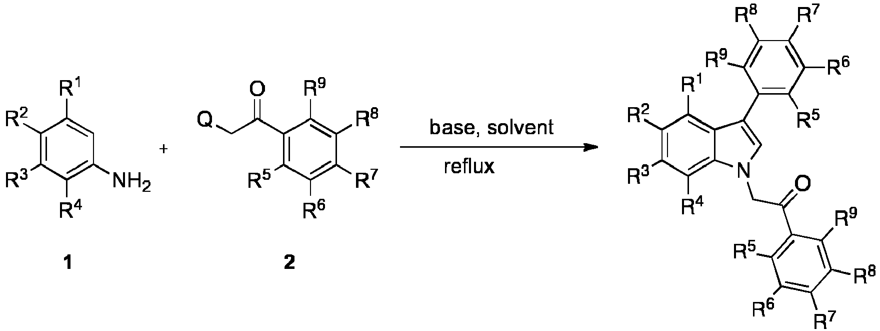

A mixture of the aniline **1** (7.0 mmol), the α-haloketone **2** (21.0 mmol) and the corresponding base (17.5 mmol) was refluxed in an appropriate solvent (70 ml) for 16-48 h. Examples of bases, solvents and reaction times employed are detailed in the examples below. The resulting mixture was cooled down and evaporated. The residue was solved in AcOEt (350 ml) and washed with HCl 1 N (3 x 100 ml). The organic fraction was dried over Na₂SO₄ and evaporated under reduced pressure. The crude product was purified by column chromatography on silicagel and/or reverse phase.

### A.2) Formation of salts

The corresponding indole (1.0 mmol) obtained following procedure A.1 was suspended in water (3.5 ml). The hydroxide of the corresponding alkaline or alkaline earth metal (1.0 mmol) was added, and the mixture was stirred for 1 h. The crude reaction solution was evaporated to dryness.

### A.3) Formation of acetylated derivatives

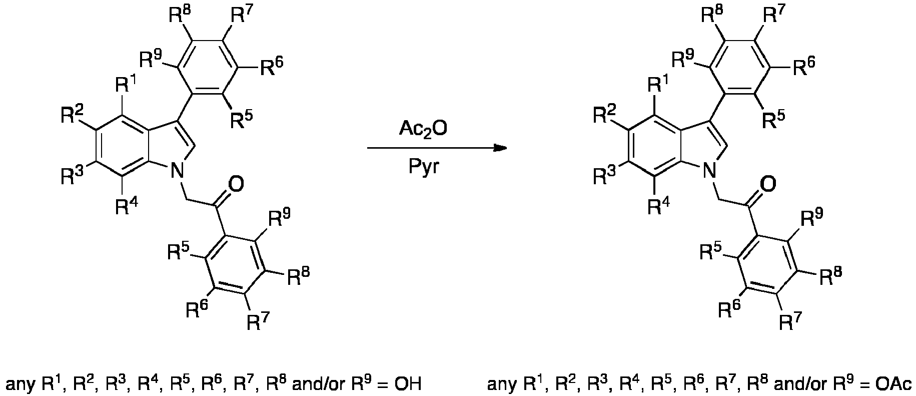

The corresponding indole (5.0 mmol) obtained following procedure A.1 was placed under argon atmosphere and cooled down to 0°C, and pyridine (25 ml) was added dropwise. When a homogeneous solution was formed, acetic anhydride (2.5 equivalents per hydroxyl group to be protected) was added dropwise while keeping temperature at 0°C. The mixture was stirred for 16 hours at room temperature and then evaporated. The residue was solved in AcOEt (100 ml) and washed with H₂O (5 x 15 ml). The organic fraction was dried over Na₂SO₄ and evaporated under reduced pressure. The crude product was purified by column chromatography on silicagel and/or reverse phase.

### Method B: Synthesis of oxime derivatives

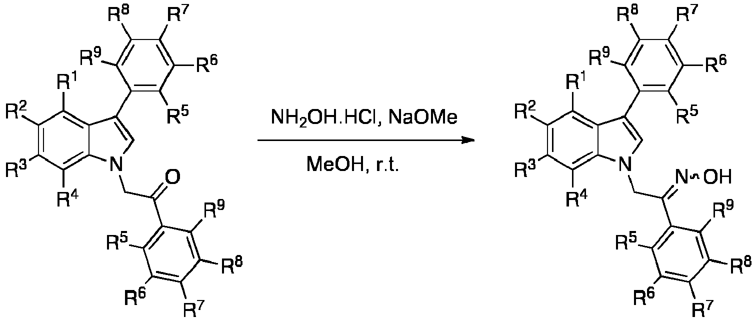

To a solution of hydroxylamine hydrochloride (0.36 g, 5.6 mmol) and phenolphtalein (1 mg) in methanol (1 ml) under inert atmosphere, an aliquot of sodium methoxide in methanol (taken from a solution of 2.70 g, 50 mmol of sodium methoxide in 10 ml of methanol) was added dropwise until a permanent pink color was observed. The corresponding indole (1 mmol) obtained following procedure A.1 and sodium methoxide in methanol (7.5 mmol, 0.75 ml of the previously prepared solution) were subsequently added, and the reaction mixture was stirred for 26h. Water (3 ml) was added, and this solution was acidified with glacial acetic acid and extracted with CH₂Cl₂ (3 x 10 ml). The combined organic fractions were dried over Na₂SO₄ and evaporated under reduced pressure.

### Method C: Synthesis of 2,2'-(arylimino)bis(1-aryl) ethanones

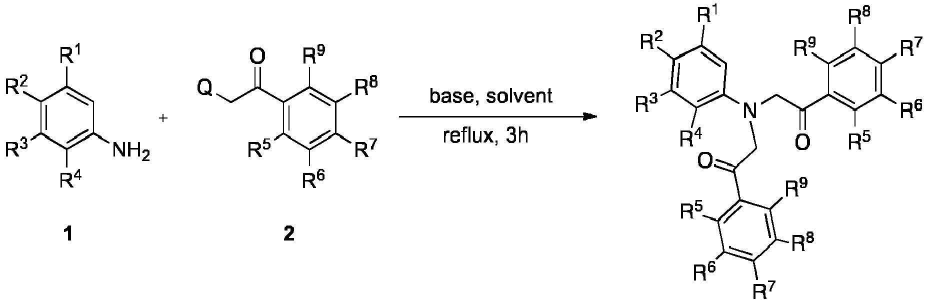

A mixture of the aniline **1** (3.0 mmol), the α-haloketone **2** (9.0 mmol) and the corresponding base (7.5 mmol) was refluxed in an appropriate solvent (30 ml) for 3 hours. Examples of bases, solvents and reaction times employed are detailed in the examples below. The resulting mixture was cooled down and evaporated. The residue was solved in AcOEt (150 ml) and washed with HCl 1 N (3 x 45 ml). The organic fraction was dried over Na₂SO₄ and evaporated under reduced pressure. The crude product was purified by column chromatography on silicagel and/or reverse phase.

### Synthesis of Compounds of the invention

### Example 1: Preparation of 1-(4-hydroxyphenyl)-2-[3-(4-hydroxyphenyl)-6-methoxy-1H-indol-1-yl]ethanone, with the following structural formula:

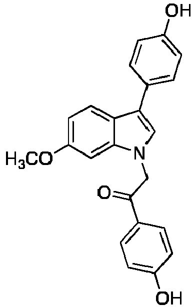

This compound was prepared following procedure A.1 from 3-methoxyaniline and 2-bromo-1-(4-hydroxyphenyl)ethanone, using N, N-dimethylaniline as base and xylene as solvent at reflux for 24 h. m.p. 198-199°C; IR 3378, 1674, 1605, 1501, 1445, 1228, 989, 829, 782 cm-¹; ¹H-NMR (500 MHz, δ ppm, CDCl₃) 9.29 (s, 1H), 7.99 (d, *J* = 8.5 Hz, 2H), 7.66 (d, *J* = 8.7 Hz, 1H), 7.42 (d, *J* = 8.3 Hz, 2H), 7.32 (s, 1H), 6.96 (s, 1H), 6.93 (d, *J* = 8.6 Hz, 2H), 6.83 (d, *J* = 8.3 Hz, 2H), 6.76 - 6.71 (m, 1H), 5.76 (s, 2H), 3.74 (s, 3H); ¹³C-NMR (75 MHz, δ ppm, DMSO-d₆) 192.2, 162.5, 155.7, 155.3, 138.4, 130.6, 130.0, 127.5, 126.4, 126.3, 125.2, 119.9, 119.7, 115.6, 115.3, 114.4, 109.3, 93.8, 55.3, 51.7. C₂₃H₁₉NO₄; MS (ESI, m/z): 372.04 [M-H]⁻.

### Example 2: Preparation of 1-(3-hydroxyphenyl)-2-[3-(3-hydroxyphenyl)-6-methoxy-1H-indol-1-yl]ethanone, with the following structural formula:

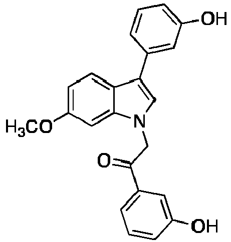

This compound was prepared following procedure A.1 from 3-methoxyaniline and 2-bromo-1-(3-hydroxyphenyl)ethanone, using *N, N*-dimethylaniline as base and xylene as solvent at reflux for 24 h. IR 3326, 1686, 1447, 1264, 1163, 776 cm⁻¹; ¹H-NMR (500 MHz, δ ppm, DMSO-d₆) 9.91 (s, 1H), 9.38 (s, 1H), 7.72 (d, J = 8.7 Hz, 1H), 7.60 (d, *J* = 7.6 Hz, 1H), 7.46 - 7.39 (m, 4H), 7.21 (t, *J* = 7.8 Hz, 1H), 7.08 - 7.02 (m, 3H), 6.96 (d, *J* = 8.3 Hz, 1H), 6.77 (dd, *J* = 8.7 Hz, *J'* = 2.1 Hz, 1H), 5.86 (s, 2H), 3.75 (s, 3H). C₂₃H₁₉NO₄; MS (ESI, m/z): 372.20 [M-H]⁻.

### Example 3: Preparation of 1-(2-hydroxyphenyl)-2-[3-(2-hydroxyphenyl)-6-methoxy-1H-indol-1-yl]ethanone, with the following structural formula:

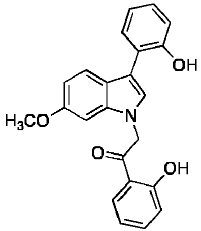

This compound was prepared following procedure A.1 from 3-methoxyaniline and 2-bromo-1-(2-hydroxyphenyl)ethanone, using *N, N*-dimethylaniline as base and xylene as solvent at reflux for 24 h. m.p. 144-146°C; IR 3440, 1654, 1450, 1258, 1158, 744 cm⁻¹; ¹H-NMR (300 MHz, δ ppm, CDCl₃) 11.31 (s, 1H), 9.37 (s, 1H), 7.97 (dd, *J* = 7.9 Hz, *J'* = 1.5 Hz, 1H), 7.61 (d, *J* = 8.8 Hz, 1H), 7.58 - 7.48 (m, 3H), 7.10 - 6.91 (m, 5H), 6.88 (dt, *J* = 7.4 Hz, *J'* = 1.1 Hz, 1H), 6.73 (dd, *J* = 8.7 Hz, *J'* = 2.2 Hz, 1H), 5.85 (s, 2H), 3.75 (s, 3H); ¹³C-NMR (126 MHz, δ ppm, DMSO-d₆) 198.2, 159.8, 155.6, 154.1, 137.7, 135.8, 130.3, 129.1, 128.5, 126.3, 122.2, 120.8, 120.6, 120.3, 119.3, 119.1, 117.6, 115.7, 111.6, 109.1, 93.6, 55.3, 53.7. C₂₃H₁₉NO₄; MS (ESI, m/z): 372.08 [M-H]⁻.

### Example 4: Preparation of 1-(3,4-dihydroxyphenyl)-2-[3-(3,4-dihydroxyphenyl)-6-methoxy-1H-indol-1-yl]ethanone, with the following structural formula:

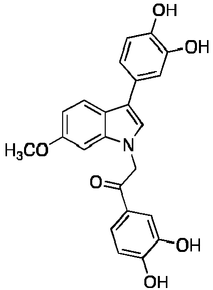

This compound was prepared following procedure A.1 from 3-methoxyaniline and 2-bromo-1-(3,4-dihydroxyphenyl)ethanone, using sodium bicarbonate as base and ethanol as solvent at reflux for 48 h. m.p. 140-141 °C; IR 3275, 1671, 1594, 1438, 1272, 1166, 778 cm⁻¹; ¹H-NMR (500 MHz, δ ppm, DMSO-d₆) 7.65 (d, *J* = 8.7 Hz, 1H), 7.55 (dd, *J* = 8.2 Hz, J' = 2.1 Hz, 1H), 7.44 (d, *J* = 1.9 Hz, 1H), 7.26 (s, 1H), 7.03 (d, *J* = 1.9 Hz, 1H), 6.93 - 6.85 (m, 4H), 6.78 (d, *J* = 8.1 Hz, 1H), 5.70 (s, 2H), 3.73 (s, 3H); ¹³C-NMR (126 MHz, δ ppm, DMSO-d₆) 192.5, 155.8, 151.3, 145.5, 143.5, 138.5, 126.9, 126.8, 125.4, 121.6, 120.0, 119.9, 117.6, 116.2, 115.8, 115.3, 114.9, 114.1, 109.3, 93.9, 55.4, 51.7. C₂₃H₁₉NO₆; MS (ESI, m/z): 404.15 [M-H]⁻.

### Example 5: Preparation of 2-[6-methoxy-3-(2,3,4-trihydroxyphenyl)-1H-indol-1-yl]-1-(2,3,4-trihydroxyphenyl)ethanone, with the following structural formula:

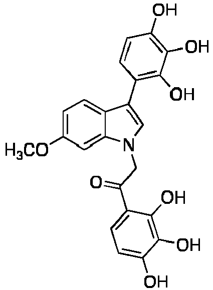

This compound was prepared following procedure A.1 from 3-methoxyaniline and 2-bromo-1-(2,3,4-trihydroxyphenyl)ethanone, using sodium bicarbonate as base and ethanol as solvent at reflux for 48 h. m.p. 132-133°C; IR 3352, 1622, 1446, 1255, 1167, 1005, 980, 789 cm⁻¹; ¹H-NMR (500 MHz, δ ppm, DMSO-d₆) 11.83 (s, 1H), 10.19 (s, 1H), 8.97 (s, 1H), 8.71 (s, 1H), 8.30 (s, 1H), 8.08 (s, 1H), 7.53 (d, *J* = 8.8 Hz, 2H), 7.33 (s, 1H), 6.94 (d, *J* = 1.8 Hz, 1H), 6.79 (d, *J* = 8.3 Hz, 1H), 6.68 (dd, J = 8.7 Hz, *J'* = 1.9 Hz, 1H), 6.51 (d, *J* = 8.6 Hz, 1H), 6.39 (d, *J* = 8.3 Hz, 1H), 5.78 (s, 2H), 3.74 (s, 3H); ¹³C-NMR (126 MHz, δ ppm, DMSO-d₆) 198.5, 155.7, 153.0, 152.9, 151.9, 144.4, 143.8, 137.9, 132.7, 127.5, 122.2, 121.3, 120.9, 119.3, 114.5, 112.5, 111.9, 108.2, 106.9, 100.4, 93.6, 55.5, 51.6. C₂₃H₁₉NO₈; MS (ESI, m/z): 436.10 [M-H]⁻.

### Example 6: Preparation of 1-(4-hydroxyphenyl)-2-[3-(4-hydroxyphenyl)-4,6-dimethoxy-1H-indol-1-yl]ethanone, with the following structural formula:

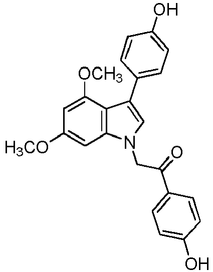

This compound was prepared following procedure A.1 from 3,5-dimethoxyaniline and 2-bromo-1-(4-hydroxyphenyl)ethanone, using *N, N-*dimethylaniline as base and xylene as solvent at reflux for 24 h. m.p. 226°C (dec.); IR 3368, 1680, 1605, 1502, 1449, 1224, 991, 837, 625 cm⁻¹; ¹H-NMR (500 MHz, δ ppm, DMSO-d₆) 10.47 (s, 1H), 9.17 (s, 1H), 7.98 (d, *J* = 8.6 Hz, 2H), 7.30 (d, *J* = 8.5 Hz, 2H), 7.00 (s, 1H), 6.92 (d, *J* = 8.6 Hz, 2H), 6.73 (d, *J* = 8.5 Hz, 2H), 6.53 (s, 1H), 6.20 (s, 1H), 5.70 (s, 2H), 3.73 (s, 3H), 3.72 (s, 3H); ¹³C-NMR (75 MHz, δ ppm, DMSO-d₆) 192.1, 162.5, 156.6, 155.2, 154.2, 139.2, 130.6, 129.9, 126.8, 126.4, 125.2, 116.5, 115.3, 114.4, 109.9, 91.5, 86.2, 55.3, 54.9, 51.8. C₂₄H₂₁NO₅; MS (ESI, m/z): 402.02 [M-H]⁻.

### Example 7: Preparation of 1-(2-hydroxyphenyl)-2-[3-(2-hydroxyphenyl)-4,6-dimethoxy-1H-indol-1-yl]ethanone, with the following structural formula:

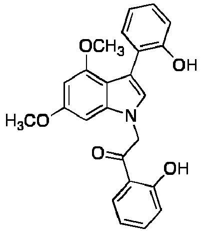

This compound was prepared following procedure A.1 from 3,5-dimethoxyaniline and 2-bromo-1-(2-hydroxyphenyl)ethanone, using N, N-dimethylaniline as base and xylene as solvent at reflux for 24 h. m.p. 150-151°C; IR 3422, 1647, 1617, 1497, 1452, 1283, 1198, 753 cm⁻¹; ¹H-NMR (500 MHz, δ ppm, CDCl₃) 11.66 (s, 1H), 7.83 (d, *J* = 8.0 Hz, 1H), 7.56 (t, *J* = 7.7 Hz, 1H), 7.31 (d, *J* = 7.4 Hz, 1H), 7.26 - 7.20 (m, 2H), 7.05 (d, *J* = 8.4 Hz, 1H), 7.02-6.96 (m, 2H), 6.94 (t, *J* = 7.4 Hz, 1H), 6.83 (s, 1H), 6.26 (d, *J* = 10.5 Hz, 2H), 5.47 (s, 2H), 3.80 (s, 3H), 3.74 (s, 3H); ¹³C-NMR (126 MHz, δ ppm, DMSO-d₆) 198.1, 159.8, 156.6, 154.8, 154.2, 138.6, 135.8, 132.3, 130.3, 127.0, 126.5, 122.9, 120.3, 119.3, 117.9, 117.6, 114.9, 111.5, 111.3, 91.7, 86.2, 55.4, 55.1, 53.8. C₂₄H₂₁NO₅; MS (ESI, m/z): 402.21 [M-H]⁻.

### Example 8: Preparation of 1-(3,4-dihydroxyphenyl)-2-[3-(3,4-dihydroxyphenyl)-4,6-dimethoxy-1H-indol-1-yl]ethanone, with the following structural formula:

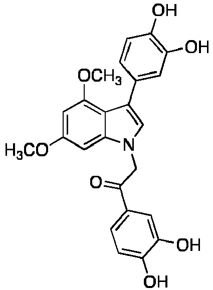

This compound was prepared following procedure A.1 from 3,5-dimethoxyaniline and 2-bromo-1-(3,4-dihydroxyphenyl)ethanone, using sodium bicarbonate as base and ethanol as solvent at reflux for 16 h. m.p. 101-103°C; IR 3346, 1668, 1595, 1448, 1269, 1164, 806 cm⁻¹; ¹H-NMR (500 MHz, δ ppm, DMSO-d₆) 9.96 (s, 1H), 9.41 (s, 1H), 8.69 (s, 1H), 8.58 (s, 1H), 7.54 (d, J = 8.1 Hz, 1H), 7.44 (s, 1H), 6.96 (s, 2H), 6.89 (d, *J* = 8.1 Hz, 1H), 6.76 (d, *J* = 8.3 Hz, 1H), 6.68 (d, *J* = 8.0 Hz, 1H), 6.50 (s, 1H), 6.19 (s, 1H), 5.65 (s, 2H), 3.74 (s, 3H), 3.71 (s, 3H); ¹³C-NMR (126 MHz, δ ppm, DMSO-d₆) 192.2, 156.6, 154.2, 151.2, 145.4, 144.3, 143.2, 139.2, 127.4, 126.8, 125.3, 121.5, 119.9, 116.9, 116.8, 115.2, 114.9, 114.9, 109.9, 91.5, 86.2, 59.8, 55.3, 55.0, 51.7. C₂₄H₂₁NO₇. MS (ESI, m/z): 434.20 [M-H]⁻.

### Example 9: Preparation of 1-(2,4-dihydroxyphenyl)-2-[3-(2,4-dihydroxyphenyl)-4,6-dimethoxy-1H-indol-1-yl]ethanone, with the following structural formula:

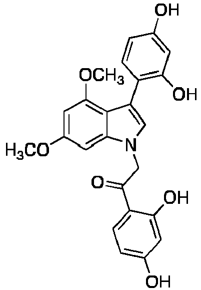

This compound was prepared following procedure A.1 from 3,5-dimethoxyaniline and 2-bromo-1-(2,4-dihydroxyphenyl)ethanone, using sodium bicarbonate as base and ethanol as solvent at reflux for 16 h. m.p. 231-232°C; IR 3392, 1698, 1631, 1501, 1456, 1224, 1146, 805 cm⁻¹; ¹H-NMR (500 MHz, δ ppm, DMSO-d₆) 11.85 (s, 1H), 10.67 (s, 1H), 9.00 (s, 1H), 8.74 (s, 1H), 7.92 (d, *J* = 8.7 Hz, 1H), 7.02 - 6.93 (m, 2H), 6.51 (s, 1H), 6.46 (d, *J* = 8.4 Hz, 1H), 6.32 (d, *J* = 8.0 Hz, 2H), 6.19 (d, *J* = 8.0 Hz, 1H), 6.15 (s, 1H), 5.67 (s, 2H), 3.71 (s, 3H), 3.65 (s, 3H); ¹³C-NMR (126 MHz, δ ppm, DMSO-d₆) 196.9, 164.8, 163.5, 156.4, 156.2, 155.6, 154.3, 138.5, 132.6, 132.5, 126.4, 113.9, 111.8, 111.7, 111.5, 108.3, 105.4, 102.5, 102.2, 91.5, 86.1, 55.3, 55.0, 51.9. C₂₄H₂₁NO₇. MS (ESI, m/z): 434.00 [M-H]⁻.

### Example 10: Preparation of 1-(3,5-dihydroxyphenyl)-2-[3-(3,5-dihydroxyphenyl) -4,6-dimethoxy-1H-indol-1-yl]ethanone, with the following structural formula:

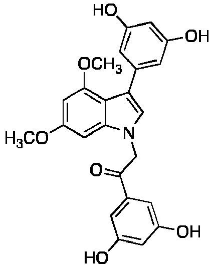

This compound was prepared following procedure A.1 from 3,5-dimethoxyaniline and 2-bromo-1-(3,5-dihydroxyphenyl)ethanone, using sodium bicarbonate as base and ethanol as solvent at reflux for 16 h. m.p. 234-235°C; IR 3408, 1698, 1598, 1456, 1360, 1205, 1161, 994, 852, 803, 688 cm⁻¹; ¹H-NMR (500 MHz, δ ppm, DMSO-d₆) 9.66 (s, 1H), 8.94 (s, 1H), 7.04 (s, 1H), 6.93 (s, 2H), 6.54 (s, 2H), 6.43 (s, 2H), 6.22 (s, 1H), 6.07 (s, 1H), 5.69 (s, 2H), 3.75 (s, 3H), 3.72 (s, 3H); ¹³C-NMR (126 MHz, δ ppm, DMSO-d₆) 193.9, 158.7, 157.5, 156.1, 154.1, 139.2, 137.5, 136.6, 126.0, 117.0, 109.7, 107.3, 106.1, 99.8, 91.8, 86.3, 68.5, 55.8, 55.3, 55.1, 52.3. C₂₄H₂₁NO₇. MS (ESI, m/z): 434.13 [M-H]⁻.

### Example 11: Preparation of 2-[4,6-dimethoxy-3-(2,3,4-trihydroxyphenyl)-1H-indol-1-yl]-1-(2,3,4-trihydroxyphenyl)ethanone, with the following structural formula:

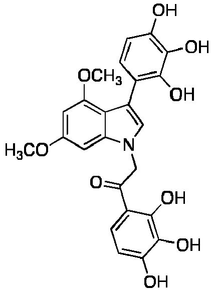

This compound was prepared following procedure A.1 from 3,5-dimethoxyaniline and 2-bromo-1-(2,3,4-trihydroxyphenyl)ethanone, using sodium bicarbonate as base and ethanol as solvent at reflux for 16 h. m.p. 166-167°C; IR 3187, 1618, 1451, 1264, 1201, 1033, 793 cm⁻¹; ¹H-NMR (500 MHz, δ ppm, DMSO-d₆) 7.51 (d, J = 8.8 Hz, 1H), 6.97 (s, 1H), 6.55 (d, J = 8.4 Hz, 1H), 6.52 (d, *J* = 1.4 Hz, 1H), 6.50 (d, *J* = 8.8 Hz, 1H), 6.27 (d, *J* = 8.3 Hz, 1H), 6.16 (d, *J* = 1.4 Hz, 1H), 5.69 (s, 2H), 3.71 (s, 3H), 3.64 (s, 3H); ¹³C-NMR (126 MHz, δ ppm, DMSO-d₆) 198.2, 156.5, 154.3, 152.7, 151.8, 145.5, 144.2, 144.1, 138.6, 132.5, 1 32.4, 126.5, 121.9, 115.0, 111.9, 111.8, 111.6, 107.9, 105.8, 91.6, 86.2, 55.4, 55.1, 51.5. C₂₄H₂₁NO₉. MS (ESI, m/z): 466.16 [M-H]⁻.

### Example 12: Preparation of 1-(4-fluorophenyl)-2-[3-(4-fluorophenyl)-4,6-dimethoxy-1H-indol-1-yl] ethanone, with the following structural formula:

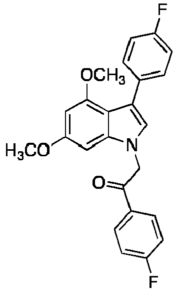

This compound was prepared following procedure A.1 from 3,5-dimethoxyaniline and 2-bromo-1-(4-fluorophenyl)ethanone, using potassium carbonate as base and ethanol as solvent at reflux for 16 h. m.p. 98-100°C; IR 3445, 1699, 1592, 1504, 1220, 1157, 1071, 833, 811 cm⁻¹; ¹H-NMR (300 MHz, δ ppm, CDCl₃) 8.03 - 7.94 (m, 2H), 7.57 - 7.48 (m, 2H), 7.15 (t, *J* = 8.6 Hz, 2H), 7.02 (t, *J* = 8.9 Hz, 2H), 6.81 (s, 1H), 6.26 (d, *J* = 1.9 Hz, 1H), 6.22 (d, *J* = 1.9 Hz, 1H), 5.32 (s, 2H), 3.78 (s, 3H), 3.77 (s, 3H); ¹³C-NMR (126 MHz, δ ppm, DMSO-d₆) 192.8, 166.4, 164.3, 161.5, 159.6, 156.9, 154.1, 139.4, 132.3, 131.2, 131.1, 130.5, 130.4, 129.9, 127.7, 126.1, 115.9, 115.8, 114.3, 114.2, 109.7, 91.9, 86.4, 55.4, 55.0, 52.4. C₂₄H₁₉F₂NO₃. MS (ESI, m/z): 406.03 [M-H]⁻.

### Example 13: Preparation of 1-(4-hydroxyphenyl)-2-[3-(4-hydroxyphenyl)-4,7-dimethoxy-1H-indol-1-yl]ethanone, with the following structural formula:

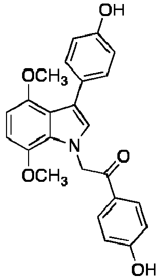

This compound was prepared following procedure A.1 from 2,5-dimethoxyaniline and 2-bromo-1-(4-hydroxyphenyl)ethanone, using N, N-dimethylaniline as base and xylene as solvent at reflux for 24 h. m.p. 240-241°C; IR 3362, 1673, 1602, 1516, 1437, 1254, 1237, 1086, 1059, 837 cm⁻¹; ¹H-NMR (500 MHz, δ ppm, DMSO-d₆) 10.42 (s, 1H), 9.17 (s, 1H), 7.93 (d, *J* = 8.7 Hz, 2H), 7.30 (d, *J* = 8.5 Hz, 2H), 7.09 (s, 1H), 6.92 (d, *J* = 8.7 Hz, 2H), 6.74 (d, *J* = 8.5 Hz, 2H), 6.50 (d, *J* = 8.5 Hz, 1H), 6.39 (d, *J* = 8.4 Hz, 1H), 5.79 (s, 2H), 3.67 (s, 3H), 3.53 (s, 3H); ¹³C-NMR (126 MHz, δ ppm, DMSO-d₆) 192.3, 162.3, 155.3, 148.3, 141.9, 130.3, 130.2, 128.2, 127.7, 126.5, 126.4, 117.7, 116.4, 115.4, 114.3, 102.8, 99.6, 55.8, 55.3, 54.5. C₂₄H₂₁NO₅. MS (ESI, m/z): 404.02 [M+1]⁺.

### Example 14: Preparation of 1-(4-hydroxyphenyl)-2-[3-(4-hydroxyphenyl)-5,6-dimethoxy-1H-indol-1-yl]ethanone, with the following structural formula:

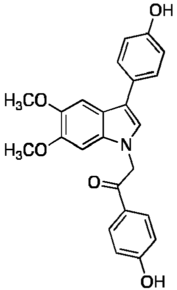

This compound was prepared following procedure A.1 from 3,4-dimethoxyaniline and 2-bromo-1-(4-hydroxyphenyl)ethanone, using *N, N-*dimethylaniline as base and xylene as solvent at reflux for 24 h. m.p. 142-143°C; IR 3416, 3371, 1673, 1579, 1488, 1208, 1165, 1071, 986, 832 cm⁻¹; ¹H-NMR (500 MHz, δ ppm, DMSO-d₆) 7.97 (d, *J* = 8.4 Hz, 2H), 7.42 (d, *J* = 8.2 Hz, 2H), 7.25 (d, *J* = 13.0 Hz, 2H), 7.03 (s, 1H), 6.90 (d, *J* = 8.1 Hz, 2H), 6.83 (d, *J* = 8.2 Hz, 2H), 5.73 (s, 2H), 3.78 (s, 3H), 3.74 (s, 3H); ¹³C-NMR (126 MHz, δ ppm, DMSO-d₆) 192.4, 162.5, 155.3, 146.7, 144.8, 132.3, 130.7, 127.5, 126.6, 126.5, 124.9, 118.2, 115.7, 115.5, 115.4, 102.0, 94.5, 56.2, 55.9, 51.9. C₂₄H₂₁NO₅. MS (ESI, m/z): 402.06 [M-H]⁻.

### Example 15: Preparation of 2-[5-hydroxy-3-(4-hydroxyphenyl)-6-methoxy-1H-indol-1-yl]-1-(4-hydroxyphenyl)ethanone, with the following structural formula:

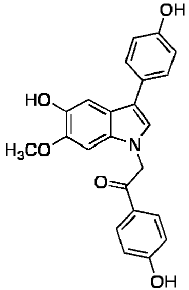

This compound was prepared following procedure A.1 from 4-amino-2-methoxyphenol and 2-bromo-1-(4-hydroxyphenyl)ethanone, using N, N-dimethylaniline as base and xylene as solvent at reflux for 24 h. m.p. 275-277 °C; IR 3471, 3362, 1668, 1594, 1483, 1439, 1340, 1215, 1165, 1065, 991, 832 cm⁻¹; ¹H-NMR (300 MHz, δ ppm, DMSO-d₆) 10.44 (s, 1H), 9.22 (s, 1H), 8.28 (s, 1H), 7.98 (d, *J* = 8.8 Hz, 2H), 7.37 (d, *J* = 8.6 Hz, 2H), 7.23 (s, 1H), 7.16 (s, 1H), 6.99 - 6.88 (m, 3H), 6.82 (d, *J* = 8.6 Hz, 2H), 5.70 (s, 2H), 3.75 (s, 3H); ¹³C-NMR (126 MHz, δ ppm, DMSO-d₆) 192.5, 162.5, 155.1, 145.6, 141.8, 131.8, 130.7, 127.2, 126.8, 126.5, 124.8, 118.8, 115.6, 115.4, 114.7, 104.0, 94.3, 55.9, 51.8. C₂₃H₁₉NO₅. MS (ESI, m/z): 388.15 [M-H]⁻.

### Example 16: Preparation of 2-[4,6-difluoro-3-(4-hydroxyphenyl)-1H-indol-1-yl]-1-(4-hydroxyphenyl) ethanone, with the following structural formula:

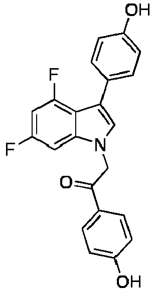

This compound was prepared following procedure A.1 from 3,5-difluoroaniline and 2-bromo-1-(4-hydroxyphenyl)ethanone, using *N, N*-dimethylaniline as base and xylene as solvent at reflux for 24 h. m.p. 234-235 °C; IR 3385, 1673, 1601, 1549, 1508, 1463, 1237, 1164, 1100, 981, 837 cm⁻¹; ¹H-NMR (500MHz, δ ppm, DMSO-d₆) 10.48 (s, 1H), 9.33 (s, 1H), 7.97 (d, *J* = 8.7 Hz, 2H), 7.37 (s, 1H), 7.33 (dd, *J* = 8.5 Hz, *J'* = 2.1 Hz, 2H), 7.20 (dd, *J* = 9.9 Hz, *J'* = 1.8 Hz, 1H), 6.93 (d, *J* = 8.7 Hz, 2H), 6.87 - 6.82 (m, 1H), 6.80 (d, *J* = 8.5 Hz, 2H), 5.81 (s, 2H); ¹³C-NMR (126 MHz, δ ppm, DMSO-d₆) 191.6, 162.6, 155.9, 130.7, 129.3, 129.3, 128.3, 127.7, 126.2, 124.9, 115.7, 115.4, 115.1, 114.9, 94.8, 93.4, 52.2. C₂₂H₁₅F₂NO₃. MS (ESI, m/z): 378.05 [M-H]⁻.

### Example 17: Preparation of 2-[6-hydroxy-3-(4-hydroxyphenyl)-1H-indol-1-yl]-1-(4-hydroxyphenyl) ethanone, with the following structural formula:

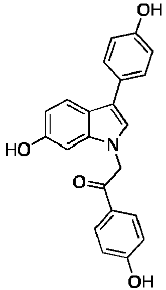

This compound was prepared following procedure A.1 from 3-hydroxyaniline and 2-bromo-1-(4-hydroxyphenyl)ethanone, using *N, N*-dimethylaniline as base and xylene as solvent at reflux for 24 h. m.p. 268°C (dec.); IR 3360, 1670, 1603, 1552, 1458, 1336, 1235, 1166, 829, 785, 589 cm⁻¹; ¹H-NMR (500 MHz, δ ppm, DMSO-d₆) 10.49 (s, 1H), 9.27 (s, 1H), 8.97 (s, 1H), 8.00 (d, *J* = 8.5 Hz, 2H), 7.59 (d, *J* = 8.5 Hz, 1H), 7.42 (d, *J* = 8.3 Hz, 2H), 7.26 (s, 1H), 6.93 (d, *J* = 8.5 Hz, 2H), 6.84 (d, *J* = 8.3 Hz, 2H), 6.63 (d, *J* = 9.4 Hz, 1H), 6.61 (s, 1H), 5.65 (s, 2H); ¹³C-NMR (126 MHz, δ ppm, DMSO-d₆) 192.4, 162.6, 155.3, 153.3, 138.6, 130.7, 127.4, 126.5, 126.4, 124.7, 119.8, 119.2, 115.6, 115.5, 115.4, 109.9, 95.5, 51.7. C₂₂H₁₇NO₄. MS (ESI, m/z): 358.17 [M-H]⁻.

### Example 18: Preparation of 2-[5-hydroxy-3-(4-hydroxyphenyl)-1H-indol-1-yl]-1-(4-hydroxyphenyl) ethanone, with the following structural formula:

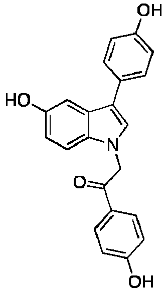

This compound was prepared following procedure A.1 from 4-hydroxyaniline and 2-bromo-1-(4-hydroxyphenyl)ethanone, using N, N-dimethylaniline as base and xylene as solvent at reflux for 24 h. m.p. 188-189°C; IR 3355, 1647, 1601, 1572, 1456, 1351, 1217, 1161, 839, 792 cm⁻¹; ¹H-NMR (500 MHz, δ ppm, DMSO-d₆) 10.58 (s, 1H), 9.36 (s, 1H), 8.82 (s, 1H), 7.97 (d, *J* = 8.7 Hz, 2H), 7.38 (d, *J* = 8.5 Hz, 2H), 7.36 (s, 1H), 7.14 (d, *J* = 1.9 Hz, 1H), 7.11 (d, *J* = 8.8 Hz, 1H), 6.91 (d, *J* = 8.7 Hz, 2H), 6.83 (d, *J* = 8.5 Hz, 2H), 6.63 (dd, *J* = 8.7 Hz, *J'* = 2.0 Hz, 1H), 5.68 (s, 2H); ¹³C-NMR (126 MHz, δ ppm, DMSO-d₆) 192.7, 162.7, 155.3, 151.4, 132.2, 130.8, 127.6, 127.3, 126.8, 126.5, 126.4, 115.8, 115.6, 114.8, 111.6, 110.8, 103.4, 55.9. C₂₂H₁₇NO₄. MS (ESI, m/z): 358.17 [M-H]⁻.

### Example 19: Preparation of 2-[5-fluoro-3-(4-hydroxyphenyl)-1H-indol-1-yl]-1-(4-hydroxyphenyl) ethanone, with the following structural formula:

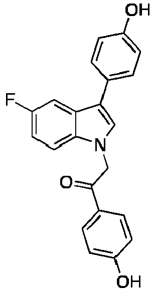

This compound was prepared following procedure A.1 from 4-fluoroaniline and 2-bromo-1-(4-hydroxyphenyl)ethanone, using N, N-dimethylaniline as base and xylene as solvent at reflux for 24 h. m.p. 236-237°C; IR 3580, 3437, 3257, 1654, 1591, 1478, 1456, 1237, 1172, 992, 878, 835, 799 cm⁻¹; ¹H-NMR (500 MHz, δ ppm, DMSO-d₆) 10.49 (s, 1H), 9.33 (s, 1H), 7.99 (d, *J* = 8.6 Hz, 2H), 7.56 (s, 1H), 7.50 (dd, *J* = 10.3 Hz, *J'* = 2.4 Hz, 1H), 7.43 (d, *J* = 8.4 Hz, 2H), 7.39 (dd, *J* = 8.9 Hz, *J'* = 4.5 Hz, 1H), 6.97 (td, *J* = 9.1 Hz, *J'* = 2.4 Hz, 1H), 6.93 (d, *J* = 8.6 Hz, 2H), 6.85 (d, *J* = 8.4 Hz, 2H), 5.82 (s, 2H); ¹³C-NMR (126 MHz, δ ppm, DMSO-d₆) 192.1, 162.6, 158.4, 156.5, 155.6, 134.2, 130.7, 129.1, 128.6, 127.6, 126.2, 125.6, 115.7, 115.4, 111.5, 111.4, 109.4, 109.2, 103.9, 103.7, 55.8. C₂₂H₁₆FNO₃. MS (ESI, m/z): 360.14 [M-H]⁻.

### Example 20: Preparation of 1-(4-hydroxyphenyl)-2-[3-(4-hydroxyphenyl)-1H-indol-1-yl]ethanone, with the following structural formula:

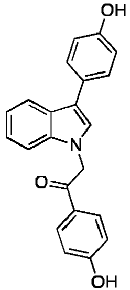

This compound was prepared following procedure A.1 from aniline and 2-bromo-1-(4-hydroxyphenyl)ethanone, using N, N-dimethylaniline as base and xylene as solvent at reflux for 24 h. m.p. 236-237°C; IR 3400, 3302, 1669, 1601, 1549, 1467, 1344, 1291, 1224, 1157, 987, 829, 664, 569 cm⁻¹; ¹H-NMR (300 MHz, δ ppm, DMSO-d₆) 10.48 (s, 1H), 9.30 (s, 1H), 7.99 (d, *J* = 8.7 Hz, 2H), 7.80 (dd, *J* = 6.7, 1.8 Hz, 1H), 7.46 (d, *J* = 9.9 Hz, 3H), 7.34 (dd, *J* = 6.9, 1.6 Hz, 1H), 7.16 - 7.05 (m, 2H), 6.93 (d, *J* = 8.7 Hz, 2H), 6.85 (d, *J* = 8.6 Hz, 2H), 5.80 (s, 2H); ¹³C-NMR (126 MHz, δ ppm, CD₃OD) 194.0, 164.3, 156.8, 139.1, 131.9, 129.4, 128.3, 128.1, 127.6, 127.5, 122.9, 120.8, 120.7, 118.0, 116.7, 116.6, 110.9, 52.8. C₂₂H₁₇NO₃. MS (ESI, m/z): 344.05 [M+1]⁺.

### Example 21: Preparation of (E)-1-(4-hydroxyphenyl)-2-[3-(4-hydroxyphenyl)-4,6-dimethoxy-1H-indol-1-yl]ethanone oxime, with the following structural formula:

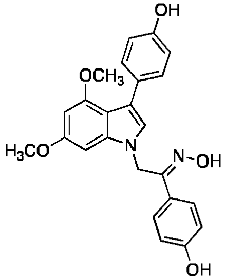

This compound was prepared following procedures A.1 and B from 3,5-dimethoxyaniline and 2-bromo-1-(4-hydroxyphenyl)ethanone, using *N, N-*dimethylaniline as base and xylene as solvent at reflux for 24 h. IR 3246, 1607, 1552, 1437, 1334, 1201, 1166, 994, 833 cm⁻¹; ¹H-NMR (500 MHz, δ ppm, DMSO-d₆) 7.48 (d, *J* = 8.4 Hz, 2H), 7.20 (d, *J* = 8.2 Hz, 2H), 6.93 (s, 1H), 6.69 (d, *J* = 8.2 Hz, 5H), 6.17 (s, 1H), 5.13 (s, 2H), 3.77 (s, 3H), 3.69 (s, 3H); ¹³C-NMR (126 MHz, δ ppm, DMSO-d₆) 157.9, 156.7, 155.3, 154.2, 150.8, 138.6, 130.3, 129.98, 126.7, 124.2, 122.0, 116.6, 114.7, 114.5, 110.1, 91.5, 86.7, 55.9, 55.4, 54.9, 49.5. C₂₄H₂₂N₂O₅. MS (ESI, m/z): 417.28 [M-H]⁻.

### Example 22: Preparation of (Z)-1-(4-hydroxyphenyl)-2-[3-(4-hydroxyphenyl)-4,6-dimethoxy-1H-indol-1-yl]ethanone oxime, with the following structural formula:

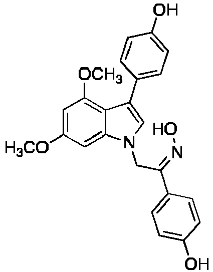

This compound was prepared following procedures A.1 and B from 3,5-dimethoxyaniline and 2-bromo-1-(4-hydroxyphenyl)ethanone, using *N, N-*dimethylaniline as base and xylene as solvent at reflux for 24 h. m.p. 189-190°C; IR 3381, 3237, 1605, 1550, 1507, 1450, 1335, 1209, 1165, 1040, 839 cm⁻¹; ¹H-NMR (500 MHz, δ ppm, DMSO-d₆) 7.46 (d, *J* = 8.3 Hz, 2H), 7.20 (d, *J* = 8.1 Hz, 2H), 7.01 (s, 1H), 6.74 - 6.61 (m, 5H), 6.16 (s, 1H), 5.36 (s, 2H), 3.75 (s, 3H), 3.67 (s, 3H); ¹³C-NMR (126 MHz, δ ppm, DMSO-d₆) 158.3, 156.8, 155.4, 154.3, 152.4, 138.2, 129.9, 127.9, 126.6, 125.3, 124.5, 116.6, 115.1, 114.5, 109.8, 91.6, 86.3, 55.3, 54.9. C₂₄H₂₂N₂O₅. MS (ESI, m/z): 417.15 [M-H]⁻.

### Example 23: Preparation of 2,2'-[(3-methoxyphenyl)azanediyl]-bis[1-(2,3,4-trihydroxyphenyl) ethanone], with the following structural formula:

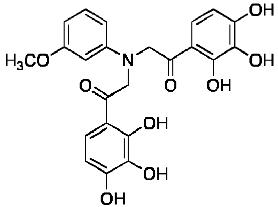

This compound was prepared following procedure C from 3-methoxyaniline and 2-bromo-1-(2,3,4-trihydroxyphenyl)ethanone, using sodium bicarbonate as base and ethanol as solvent at reflux for 3 h. m.p. 119-120°C; IR 3379, 1611, 1500, 1442, 1249, 1201, 1166, 1004, 790 cm⁻¹; ¹H-NMR (500 MHz, δ ppm, DMSO-d₆) 12.04 (s, 2H), 10.15 (s, 2H), 8.66 (s, 2H), 7.44 (d, *J* = 8.9 Hz, 2H), 6.98 (t, *J* = 8.2 Hz, 1H), 6.43 (d, *J* = 8.8 Hz, 2H), 6.21 (dd, J = 8.1 Hz, *J' =* 1.8 Hz, 1H), 6.06 (dd, *J* = 8.3 Hz, *J'* = 2.0 Hz, 1H), 5.96 (s, 1H), 4.92 (s, 4H), 3.60 (s, 3H); ¹³C-NMR (126 MHz, δ ppm, DMSO-d₆) 201.1, 160.2, 152.5, 151.8, 149.9, 132.5, 129.7, 121.51, 112.1, 107.9, 104.8, 101.2, 98.3, 57.1, 54.7. C₂₃H₂₁NO₉. MS (ESI, m/z): 454.22 [M-H]⁻.

### Example 24: Preparation of 2,2'-[(3,5-dimethoxyphenyl)azanediyl]-bis[1-(2,3,4-trihydroxyphenyl) ethanone], with the following structural formula:

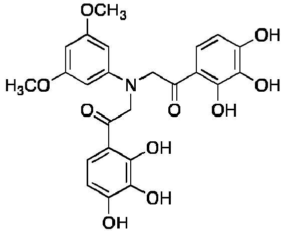

This compound was prepared following procedure C from 3,5-dimethoxyaniline and 2-bromo-1-(2,3,4-trihydroxyphenyl)ethanone, using sodium bicarbonate as base and ethanol as solvent at reflux for 3 h. IR 3422, 1620, 1452, 1295, 1250, 1201, 1168, 992, 805, 792 cm⁻¹; ¹H-NMR (500 MHz, δ ppm, DMSO-d₆) 7.43 (d, *J* = 8.8 Hz, 2H), 6.43 (d, *J* = 8.8 Hz, 2H), 5.84 (s, 1H), 5.62 (s, 2H), 4.90 (s, 4H), 3.59 (s, 6H); ¹³C-NMR (126 MHz, δ ppm, DMSO-d₆) 201.1, 161.1, 152.5, 151.7, 150.3, 132.4, 121.5, 112.1, 107.8, 91.3, 88.3, 57.1, 54.8. C₂₄H₂₃NO₁₀. MS (ESI, m/z): 484.10 [M-H]⁻.

### Example 25: Preparation of a potassium phenoxide salt of 1-(4-hydroxyphenyl)-2-[3-(4-hydroxyphenyl)-6-methoxy-1H-indol-1-yl]ethanone:

This compound was prepared following procedures A.1 and A.2 from 3-methoxyaniline and 2-bromo-1-(4-hydroxyphenyl)ethanone, using *N, N-*dimethylaniline as base and xylene as solvent at reflux for 24 h. m.p. 250°C (dec); IR 3247, 1596, 1503, 1371, 1228, 1160, 1007, 830 cm⁻¹; ¹H-NMR (500 MHz, δ ppm, DMSO-d₆) 8.56 (s, 1H), 7.87 (d, *J* = 8.3 Hz, 2H), 7.66 (d, *J* = 8.7 Hz, 1H), 7.42 (d, *J* = 8.1 Hz, 2H), 7.31 (s, 1H), 6.94 (s, 1H), 6.84 (d, *J* = 8.1 Hz, 2H), 6.74 (d, *J* = 8.4 Hz, 2H), 6.65 (d, *J* = 8.2 Hz, 1H), 5.67 (s, 2H), 3.74 (s, 3H); ¹³C-NMR (126 MHz, δ ppm, DMSO-d₆) 191.1, 165.6, 155.7, 155.6, 138.4, 130.7, 127.4, 126.8, 126.2, 125.4, 119.8, 119.7, 116.5, 115.7, 114.4, 109.2, 93.8, 55.3, 51.3. C₂₃H₁₈KNO₄.

### Example 26: Preparation of a potassium phenoxide salt of 1-(4-hydroxyphenyl)-2-[3-(4-hydroxyphenyl)-4,6-dimethoxy-1H-indol-1-yl] ethanone:

This compound was prepared following procedures A.1 and A.2 from 3,5-dimethoxyaniline and 2-bromo-1-(4-hydroxyphenyl)ethanone, using *N, N-*dimethylaniline as base and xylene as solvent at reflux for 24 h. m.p. 214-216°C; IR 3049, 1574, 1503, 1217, 1199, 1159, 1043, 832 cm⁻¹; ¹H-NMR (500 MHz, δ ppm, DMSO-d₆) 7.79 (d, *J* = 8.5 Hz, 2H), 7.30 (d, *J* = 8.1 Hz, 2H), 6.99 (s, 1H), 6.73 (d, *J* = 8.1 Hz, 2H), 6.56 (d, *J* = 8.5 Hz, 2H), 6.49 (s, 1H), 6.19 (s, 1H), 5.55 (s, 2H), 3.73 (s, 3H), 3.72 (s, 3H); ¹³C-NMR (126 MHz, δ ppm, DMSO-d₆) 190.1, 171.2, 156.5, 155.6, 154.2, 139.2, 130.8, 129.8, 126.6, 125.4, 120.6, 117.2, 116.3, 114.5, 113.8, 109.9, 91.4, 86.2, 55.3, 54.9, 51.2. C₂₄H₂₀KNO₅.

### Example 27: Preparation of a calcium phenoxide salt of 1-(4-hydroxyphenyl)-2-[3-(4-hydroxyphenyl)-4,6-dimethoxy-1H-indol-1-yl] ethanone:

This compound was prepared following procedures A.1 and A.2 from 3,5-dimethoxyaniline and 2-bromo-1-(4-hydroxyphenyl)ethanone, using N, N-dimethylaniline as base and xylene as solvent at reflux for 24 h. m.p. 182°C (dec); IR 3309, 1578, 1502, 1443, 1217, 1164, 1045, 835 cm⁻¹; ¹H-NMR (500 MHz, δ ppm, DMSO-d₆) 7.92 (d, J = 8.4 Hz, 2H), 7.30 (d, J = 8.3 Hz, 2H), 7.00 (s, 1H), 6.82 (d, J = 7.9 Hz, 2H), 6.73 (d, J = 8.4 Hz, 2H), 6.52 (s, 1H), 6.20 (s, 1H), 5.65 (s, 2H), 3.73 (s, 3H), 3.71 (s, 3H); ¹³C-NMR (126 MHz, δ ppm, DMSO-d₆) 191.6, 169.9, 156.6, 155.2, 154.2, 139.2, 130.7, 129.9, 126.8, 125.3, 116.5, 115.9, 115.4, 114.4, 109.9, 91.5, 86.2, 55.3, 54.9, 51.6. C₂₄H₂₀CaNO₅.

### Example 28: Preparation of 4-{1-[2-(4-acetoxyphenyl)-2-oxoethyl]-6-methoxy-1H-indol-3-yl}phenyl acetate, with the following structural formula:

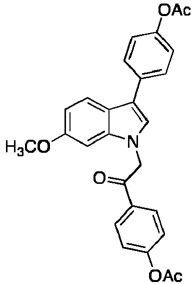

This compound was prepared following procedures A.1 and A.3 from 3-methoxyaniline and 2-bromo-1-(4-hydroxyphenyl)ethanone, using *N, N-*dimethylaniline as base and xylene as solvent at reflux for 24 h. m.p. 67-68°C; IR 1754, 1694, 1599, 1504, 1462, 1368, 1189, 1160, 1013, 909, 848 cm⁻¹; ¹H-NMR (500 MHz, δ ppm, DMSO-d₆) 8.18 (d, *J* = 8.6 Hz, 2H), 7.75 (d, *J* = 8.7 Hz, 1H), 7.66 (d, *J* = 8.5 Hz, 2H), 7.52 (s, 1H), 7.39 (d, *J* = 8.6 Hz, 2H), 7.18 (d, *J* = 8.6 Hz, 2H), 7.07 (d, *J* = 2.0 Hz, 1H), 6.78 (dd, *J* = 8.7, 2.1 Hz, 1H), 5.92 (s, 2H), 3.75 (s, 3H), 2.33 (s, 3H), 2.29 (s, 3H); ¹³C-NMR (126 MHz, δ ppm, DMSO-d₆) 193.2, 169.3, 168.8, 156.0, 154.6, 148.2, 138.6, 133.1, 132.4, 129.9, 127.0, 126.5, 122.3, 122.2, 119.7, 119.5, 114.7, 109.9, 94.0, 55.8, 55.4, 52.3, 20.9. C₂₇H₂₃NO₆. MS (ESI, m/z): 458.29 [M+1]⁺.

### Example 29: Preparation of 4-{1-[2-(4-acetoxyphenyl)-2-oxoethyl]-4,6-dimethoxy-1H-indol-3-yl} phenyl acetate, with the following structural formula:

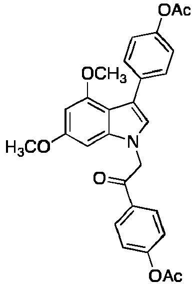

This compound was prepared following procedures A.1 and A.3 from 3,5-dimethoxyaniline and 2-bromo-1-(4-hydroxyphenyl)ethanone, using N, N-dimethylaniline as base and xylene as solvent at reflux for 24 h. m.p. 180-181°C; IR 1755, 1695, 1552, 1501, 1366, 1217, 1197, 1160, 910, 851, 810 cm-¹; ¹H-NMR (500 MHz, δ ppm, DMSO-d₆) 8.17 (d, *J* = 8.4 Hz, 2H), 7.54 (d, *J* = 8.3 Hz, 2H), 7.38 (d, *J* = 8.3 Hz, 2H), 7.19 (s, 1H), 7.09 (d, J = 8.3 Hz, 2H), 6.64 (s, 1H), 6.26 (s, 1H), 5.86 (s, 2H), 3.77 (s, 3H), 3.73 (s, 3H), 2.33 (s, 3H), 2.28 (s, 3H); ¹³C-NMR (126 MHz, δ ppm, DMSO-d₆) 193.1, 169.3, 168.8, 156.9, 154.5, 154.0, 148.3, 139.4, 133.5, 132.4, 129.8, 129.5, 126.2, 122.3, 120.8, 115.8, 109.6, 91.9, 86.4, 55.4, 55.0, 52.4, 20.9; C₂₈H₂₅NO₇. MS (ESI, m/z): 486.25 [M-H]⁻.

### Example 30: Preparation of 4-{1-[2-(3,4-diacetoxyphenyl)-2-oxoethyl]-4,6-dimethoxy-1H-indol-3-yl}-1,2-phenylene diacetate, with the following structural formula:

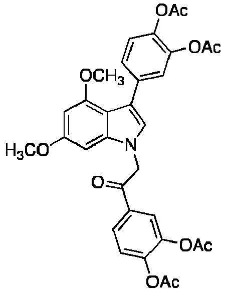

This compound was prepared following procedures A.1 and A.3 from 3,5-dimethoxyaniline and 2-bromo-1-(3,4-dihydroxyphenyl)ethanone, using sodium bicarbonate as base and ethanol as solvent at reflux for 16 h. m.p. 72-73°C; IR 1766, 1698, 1605, 1503, 1369, 1257, 1196, 1154, 1008, 899, 819 cm⁻¹; ¹H-NMR (500 MHz, δ ppm, DMSO-d₆) 8.08 (dd, *J* = 8.4 Hz, *J*' = 1.9 Hz, 1H), 7.98 (d, *J* = 1.9 Hz, 1H), 7.54 (d, *J* = 8.4 Hz, 1H), 7.42 (dd, J = 8.4 Hz, *J*' = 2.0 Hz, 1H), 7.38 (d, *J* = 1.9 Hz, 1H), 7.24 (s, 1H), 7.20 (d, *J* = 8.4 Hz, 1H), 6.67 (d, *J* = 1.6 Hz, 1H), 6.26 (d, J = 1.6 Hz, 1H), 5.86 (s, 2H), 3.77 (s, 3H), 3.73 (s, 3H), 2.33 (s, 6H), 2.29 (s, 6H); ¹³C-NMR (126 MHz, δ ppm, DMSO-d₆) 192.5, 168.4, 168.3, 168.2, 167.9, 157.0, 153.9, 146.3, 142.3, 141.2, 139.6, 139.5, 134.6, 133.3, 126.9, 126.6, 126.4, 124.2, 123.6, 123.5, 122.5, 115.1, 109.3, 92.0, 86.5, 55.4, 54.9, 52.5, 20.4; C₃₂H₂₉NO₁₁. MS (ESI, m/z): 602.17 [M-H]⁻.

### Example 31: Preparation of 4-{1-[2-(3,4-diacetoxyphenyl)-2-oxoethyl]-6-methoxy-1H-indol-3-yl}-1,2-phenylene diacetate, with the following structural formula:

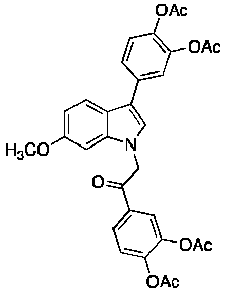

This compound was prepared following procedures A.1 and A.3 from 3-methoxyaniline and 2-bromo-1-(3,4-dihydroxyphenyl)ethanone, using sodium bicarbonate as base and ethanol as solvent at reflux for 48 h. m.p. 62-63°C; IR 1764, 1702, 1608, 1499, 1368, 1193, 1105, 1007, 890 cm⁻¹; ¹H-NMR (500 MHz, δ ppm, DMSO-d₆) 8.10 (dd, *J* = 8.3 Hz, *J'* = 1.9 Hz, 1H), 8.00 (d, *J* = 1.8 Hz, 1H), 7.74 (d, *J* = 8.8 Hz, 1H), 7.58 - 7.52 (m, 3H), 7.50 (d, *J* = 1.8 Hz, 1H), 7.30 (d, *J* = 8.4 Hz, 1H), 7.11 (d, *J* = 1.9 Hz, 1H), 6.80 (dd, *J* = 8.7 Hz, *J'* = 2.0 Hz, 1H), 5.92 (s, 2H), 3.76 (s, 3H), 2.34 (s, 6H), 2.30 (s, 6H); ¹³C-NMR (126 MHz, δ ppm, DMSO-d₆) 192.6, 168.4, 168.3, 168.2, 167.9, 156.1, 146.4, 142.3, 139.5, 138.7, 134.2, 133.3, 126.9, 124.2, 124.0, 123.9, 123.5, 120.9, 119.6, 119.3, 114.0, 110.2, 94.1, 64.9, 55.4, 52.4, 20.5, 20.4, 20.3. C₃₁H₂₇NO₁₀. MS (ESI, m/z): 572.11 [M-H]⁻.

### Biological Assays

### Example 34: In vitro biological activity

The inhibition of the enzymatic activity of DNMT1 was tested using low volume radioisotope-based assay which uses tritium-labeled AdoMet (³H-SAM) as a methyl donor. Inhibitors diluted in DMSO were added by using acoustic technology (Echo550, Labcyte Inc. Sunnyvale, CA) into enzyme/substrate mixture in nano-liter range. The reaction was initiated by the addition of ³H-SAM, and incubated at 30°C for 1 hour. Total final methylations on the substrate poly(dI-dC) were detected by a filter binding approach. Data analysis was performed using GraphPad Prism software (La Jolla, CA) for IC50 curve fits. Reactions were carried out at 1 µM of S-adenosyl-L-methionine (SAM), 25 nM DNMT1 (Human DNMT1 GenBank Accession No. NM_001130823, a-a 2-1632, with N-terminal GST tag, Mw=211 kDa, expressed in baculovirus expression system), 0.005 mg/ml Poly(dI-dC) (Sigma, Cat. # P4929). S-adenosyl-L-homocysteine (SAH) was used as standard positive control. Inhibitors were tested in 10-dose IC₅₀ (effective concentration to inhibit DNMT1 activity by 50%) with three-fold serial dilution.

| **Compound** | **IC50 (µM)** | **Compound** | **IC50 (µM)** |
|---|---|---|---|
| SAH | 0.25 | Example 14 IKDN30 | 43.35 |
| Example 1 IKDN05 | 73.12 | Example 15 IKDN31 | 576.20 |
| Example 2 IKDN33 | 30.57 | Example 16 IKDN25 | 22.52 |
| Example 3 IKDN32 | 56.67 | Example 17 IKDN37 | 50.91 |
| Example 4 IKDN43 | 13.81 | Example 18 IKDN23 | 32.95 |
| Example 5 IKDN34 | 3.53 | Example 19 IKDN24 | 59.09 |
| Example 6 IKDN16 | 27.90 | Example 20 IKDN22 | 241.45 |
| Example 7 IKDN17 | 32.86 | Example 21 IKDN35 | 103.59 |
| Example 8 IKDN42 | 6.85 | Example 22 IKDN36 | 22.48 |
| Example 9 IKDN20 | 181.25 | Example 23 IKDN41 | 27.37 |
| Example 10 IKDN19 | 44.56 | Example 24 IKDN21 | 15.55 |
| Example 11 IKDN18 | 39.44 | Example 25 IKDN38 | 19.53 |
| Example 12 IKDN27 | 103.64 | Example 26 IKDN39 | 40.42 |
| Example 13 IKDN26 | 98.40 | Example 27 IKDN40 | 46.76 |

## Claims

1. A compound of general formula (I), 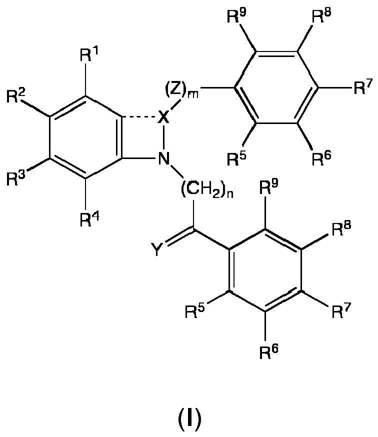 wherein:
X is a -(CH₂)ₙ- group or a -CH=CH- group;
Y is a O atom or a N-OH group;
Z is a C=Y group;
n=1-6
m is 0 or 1;
R¹-R⁹ represent, independently of each other, hydrogen, halogen, hydroxyl, alkoxyl or -OC(O)-alkyl
with the proviso that:
when X is a -(CH₂)ₙ- group, m=1 and the dashed line does not represent a bond; and
when X is a -CH=CH- group, m=0 and the dashed line represents a carbon-carbon single bond, forming together with the benzyl group an indole ring;
or a solvate or a salt or prodrug thereof;
for its use as a medicament.

2. Compound of formula (I) as defined in claim 1 for its use in the treatment of a disease or condition selected from the group consisting of cancer, hematological malignancy, proliferative diseases, genetic diseases, neurological disorders and immunological disorders.

3. A compound of formula (I): 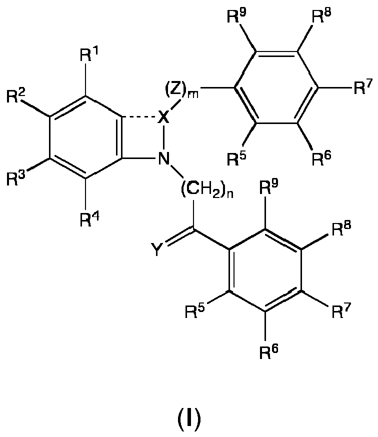 wherein:
X is a -(CH₂)ₙ- group or a -CH=CH- group;
Z is a C=Y group;
Y is a O atom or a N-OH group;
n=1-6
m is 0 or 1;
R¹-R⁹ represent, independently of each other, hydrogen, halogen, hydroxyl, alkoxyl or-OC(O)-alkyl,
with the proviso that:
when X is a -(CH₂)ₙ- group, m=1 and the dashed line does not represent a bond; and
when X is a -CH=CH- group, m=0 and the dashed line represents a carbon-carbon single bond, forming together with the benzyl group an indole ring;
or a solvate or a salt or prodrug thereof;
the following compounds being excluded when X is a -CH=CH- group:
R¹-R⁹=H Y=O and n=1;
R²=R⁴-R⁹=H, R¹=R³=OMe, Y=O and n=1;
R²=R⁴=R⁵=R⁶=R⁸=R⁹ =H; R¹=R³=OMe R⁷=Br, Y=O and n=1;
R²=R⁴=R⁵=R⁶=R⁸=R⁹=H, R¹=R³=OMe, R⁷=Cl, Y=O and n=1;
and the following compounds being excluded when X is a -(CH₂)ₙ- group:
R¹-R⁹=H Y=N-OH and n=1;
R¹-R⁹=H Y=O and n=1;
R²=OMe; R¹, R³-R⁹=H, Y=N-OH and n=1;
R²=R⁷=OMe. R¹, R³-R⁶, R⁸-R⁹=H Y=N-OH and n=1;
R²=OMe; R⁷=Cl; R¹, R³-R⁶, R⁸-R⁹=H, Y=N-OH and n=1;
R⁷=OMe; R¹-R⁶, R⁸-R⁹=H, Y=O and n=1;
R⁷=Cl; R¹-R⁶, R⁸-R⁹=H, Y=O and n=1;
R¹=R³=OMe, R²=R⁴-R⁹=H, Y=O and n=1
R¹=R³=OMe R⁷=Br, R²=R⁴=R⁵=R⁶=R⁸=R⁹ =H, Y=O and n=1;
R²=OMe; R¹, R³-R⁹=H, Y=O and n=1;
R⁴=OH; R¹-R³, R⁵-R⁹=H, Y=O and n=1;
R²=R⁷=OH, R¹, R³-R⁶, R⁸-R⁹=H, Y=O and n=1.
R¹-R⁹=H, Y=O and n=2;
R²=OMe; R¹ R³-R⁹=H, Y=O and n=2;
R²=OMe, R⁷=Cl R¹, R³-R⁶, R⁸-R⁹=H, Y=O and n=2;
R²=OMe, R⁷=Br, R¹, R³-R⁶, R⁸-R⁹=H, Y=O and n=2;
R²=R⁷=OMe, R¹, R³-R⁶, R⁸-R⁹=H, Y=O and n=2;
R²=Cl; R¹, R³-R⁹=H, Y=O and n=2;
R²=R⁷=Cl, R¹, R³-R⁶, R⁸-R⁹=H, Y=O and n=2;
R²=Cl, R⁷=OMe R¹, R³-R⁶, R⁸-R⁹=H, Y=O and n=2;
R²=Cl, R⁷=Br, R¹, R³-R⁶, R⁸-R⁹=H, Y=O and n=2;
R²=Br; R¹, R³-R⁹=H, Y=O and n=2;
R²=Br, R⁷=Cl, R¹, R³-R⁶, R⁸-R⁹=H Y=O and n=2;
R²=Br, R⁷=OMe, R¹, R³-R⁶, R⁸-R⁹=H, Y=O and n=2;
R²=R⁷=Br, R¹, R³-R⁶, R⁸-R⁹=H, Y=O and n=2.

4. Compound according to claim 3, wherein at least one of R¹, R², R³ and R⁴ is not hydrogen, and at least one of R⁵, R⁶, R⁷, R⁸and R⁹ is not hydrogen.

5. Compound according to anyone of claims 3 and 4, wherein at least one of R¹, R², R³ or R⁴ is an alkoxyl and wherein at least one of R⁵, R⁶, R⁷, R⁸ and R⁹ is a hydroxyl group.

6. Compound according to anyone of claims 3 to 5, wherein at least one of R¹, R², R³ and R⁴ is an alkoxyl and wherein at least one of R⁵ R⁶, R⁷, R⁸ and R⁹ is -OC(O)-alkyl.

7. Compound according to anyone of claims 3 to 5, wherein at least one of R¹, R², R³ and R⁴ is an alkoxyl and wherein at least one of R⁵, R⁶, R⁷, R⁸ and R⁹ is a halogen.

8. Compound according to anyone of claims 3 to 7, wherein each alkoxyl group is independently selected from -O-C₁-C₃alkyl.

9. Compound according to claim 5, wherein Y is a N-OH group.

10. Compound of general formula (I) according to anyone of claims 3 to 4 selected from the group consisting of:
[1] 1-(4-Hydroxyphenyl)-2-[3-(4-hydroxyphenyl)-6-methoxy-1*H*-indol-1-yl] ethanone, with the following structural formula: 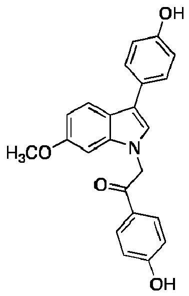
[2] 1-(3-Hydroxyphenyl)-2-[3-(3-hydroxyphenyl)-6-methoxy-1*H*-indol-1-yl] ethanone, with the following structural formula: 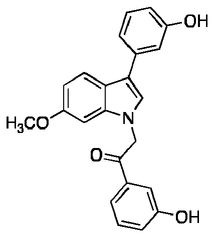
[3] 1-(2-Hydroxyphenyl)-2-[3-(2-hydroxyphenyl)-6-methoxy-1*H*-indol-1-yl] ethanone, with the following structural formula: 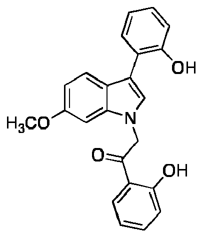
[4] 1-(3,4-Dihydroxyphenyl)-2-[3-(3,4-dihydroxyphenyl)-6-methoxy-1*H*-indol-1-yl]ethanone, with the following structural formula: 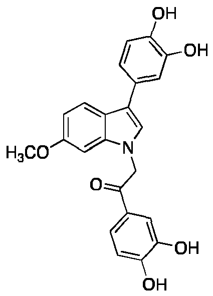
[5] 2-[6-Methoxy-3-(2,3,4-trihydroxyphenyl)-1*H*-indol-1-yl]-1-(2,3,4-trihydroxyphenyl)ethanone, with the following structural formula: 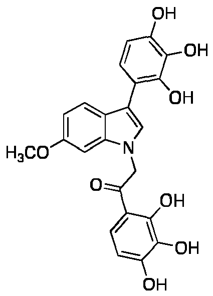
[6] 1-(4-Hydroxyphenyl)-2-[3-(4-hydroxyphenyl)-4,6-dimethoxy-1*H*-indol-1-yl] ethanone, with the following structural formula: 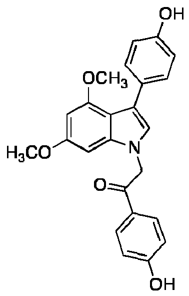
[7] 1-(2-Hydroxyphenyl)-2-[3-(2-hydroxyphenyl)-4,6-dimethoxy-1*H*-indol-1-yl] ethanone, with the following structural formula: 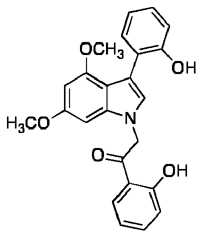
[8] 1-(3,4-Dihydroxyphenyl)-2-[3-(3,4-dihydroxyphenyl)-4,6-dimethoxy-1*H-*indol-1-yl]ethanone, with the following structural formula: 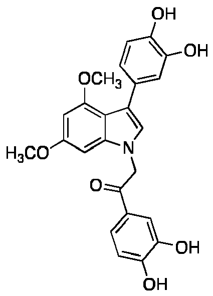
[9] 1-(2,4-Dihydroxyphenyl)-2-[3-(2,4-dihydroxyphenyl)-4,6-dimethoxy-1*H-*indol-1-yl]ethanone, with the following structural formula: 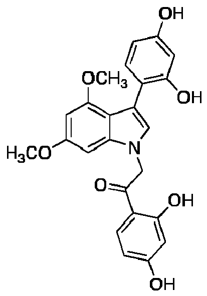
[10] 1-(3,5-dihydroxyphenyl)-2-(3-(3,5-dihydroxyphenyl)-4,6-dimethoxy-1*H-*indol-1-yl)ethanone, with the following structural formula: 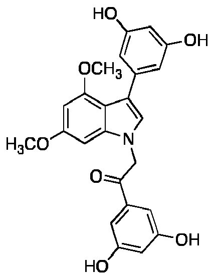
[11] 2-[4,6-Dimethoxy-3-(2,3,4-trihydroxyphenyl)-1*H*-indol-1-yl]-1-(2,3,4-trihydroxyphenyl)ethanone, with the following structural formula: 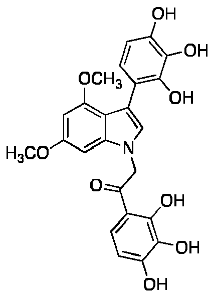
[12] 1-(4-Fluorophenyl)-2-[3-(4-fluorophenyl)-4,6-dimethoxy-1*H*-indol-1-yl] ethanone, with the following structural formula: 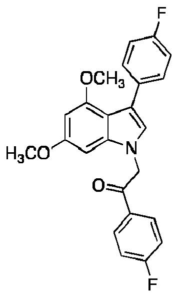
[13] 1-(4-Hydroxyphenyl)-2-[3-(4-hydroxyphenyl)-4,7-dimethoxy-1*H*-indol-1-yl]ethanone, with the following structural formula: 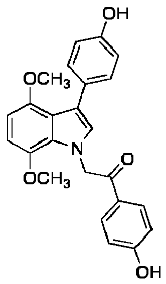
[14] 1-(4-Hydroxyphenyl)-2-[3-(4-hydroxyphenyl)-5,6-dimethoxy-1*H*-indol-1-yl]ethanone, with the following structural formula: 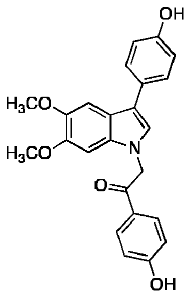
[15] 2-[5-Hydroxy-3-(4-hydroxyphenyl)-6-methoxy-1*H*-indol-1-yl]-1-(4-hydroxyphenyl)ethanone, with the following structural formula: 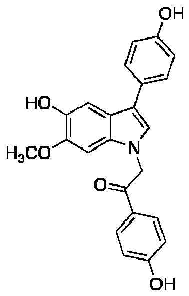
[16] 2-[4,6-Difluoro-3-(4-hydroxyphenyl)-1*H*-indol-1-yl]-1-(4-hydroxyphenyl) ethanone, with the following structural formula: 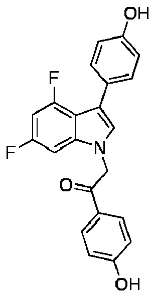
[17] 2-[6-Hydroxy-3-(4-hydroxyphenyl)-1*H*-indol-1-yl]-1-(4-hydroxyphenyl) ethanone, with the following structural formula: 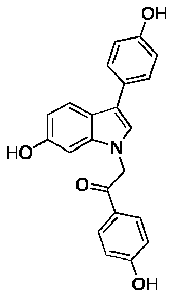
[18] 2-[5-Hydroxy-3-(4-hydroxyphenyl)-1*H*-indol-1-yl]-1-(4-hydroxyphenyl) ethanone, with the following structural formula: 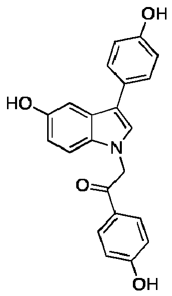
[19] 2-[5-Fluoro-3-(4-hydroxyphenyl)-1*H*-indol-1-yl]-1-(4-hydroxyphenyl) ethanone, with the following structural formula: 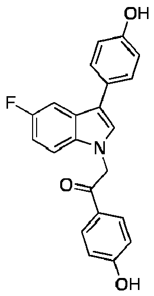
[20] 1-(4-Hydroxyphenyl)-2-[3-(4-hydroxyphenyl)-1*H*-indol-1-yl]ethanone, with the following structural formula: 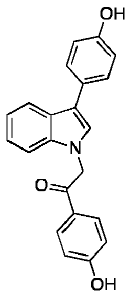
[21] ((*E*)-1-(4-Hydroxyphenyl)-2-[3-(4-hydroxyphenyl)-4,6-dimethoxy-1H-indol-1-yl]ethanone oxime, with the following structural formula: 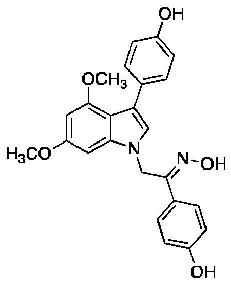
[22] ((Z)-1-(4-Hydroxyphenyl)-2-[3-(4-hydroxyphenyl)-4,6-dimethoxy-1H-indol-1-yl]ethanone oxime, with the following structural formula: 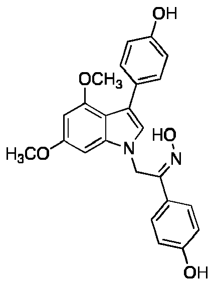
[23] 2,2'-[(3-Methoxyphenyl)azanediyl]-bis[1-(2,3,4-trihydroxyphenyl) ethanone], with the following structural formula: 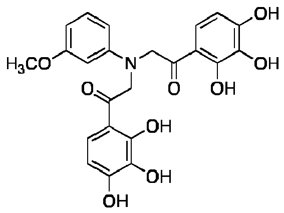
[24] 2,2'-[(3,5-dimethoxyphenyl)azanediyl]-bis[1-(2,3,4-trihydroxyphenyl) ethanone], with the following structural formula: 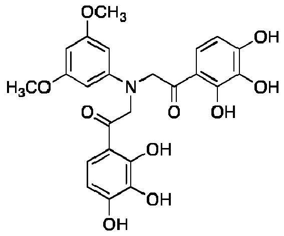
[25] Potassium salt of 1-(4-hydroxyphenyl)-2-[3-(4-hydroxyphenyl)-6-methoxy-1*H*-indol-1-yl] ethanone
[26] Potassium salt of 1-(4-hydroxyphenyl)-2-[3-(4-hydroxyphenyl)-4,6-dimethoxy-1*H*-indol-1-yl] ethanone
[27] Calcium salt of 1-(4-hydroxyphenyl)-2-[3-(4-hydroxyphenyl)-4,6-dimethoxy-1*H*-indol-1-yl] ethanone
[28] 4-{1-[2-(4-Acetoxyphenyl)-2-oxoethyl]-6-methoxy-1H-indol-3-yl}phenyl acetate, with the following structural formula: 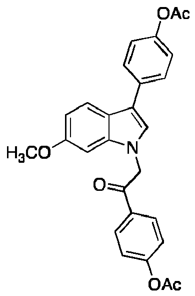
[29] 4-{1-[2-(4-Acetoxyphenyl)-2-oxoethyl]-4,6-dimethoxy-1*H*-indol-3-yl} phenyl acetate, with the following structural formula: 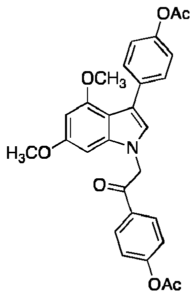
[30] 4-{1-[2-(3,4-Diacetoxyphenyl)-2-oxoethyl]-4,6-dimethoxy-1*H*-indol-3-yl}-1,2-phenylene diacetate, with the following structural formula: 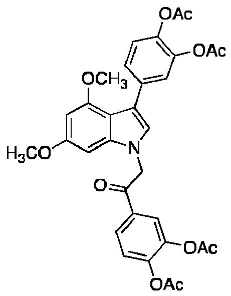
[31] 4-{1-[2-(3,4-Diacetoxyphenyl)-2-oxoethyl]-6-methoxy-1*H*-indol-3-yl}-1,2-phenylene diacetate, with the following structural formula: 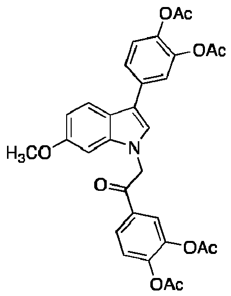
or a solvate or a salt or prodrug thereof.

11. Process for the preparation of a compound of general formula (I) as defined in claim 3, wherein R¹-R⁹ and n have the meaning given in claim 3, X is - CH=CH-, Y=O and m=0, which comprises reacting:
a) a compound of general formula (II): 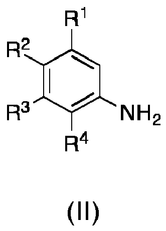 wherein R¹, R², R³ and R⁴ have the meaning given above;
with
b) a compound of general formula (III), 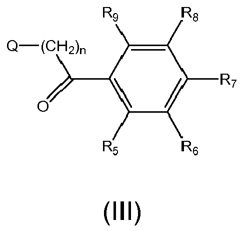 wherein Q can be a chlorine, bromine or iodine atom, or a leaving group, and R⁵, R⁶, R⁷, R⁸, R⁹ and n have the meaning given above;
in the presence of
c) a base, either organic or inorganic; and
d) a solvent
leaving the reaction to react for at least 16 hours,
and, optionally, performing one or more of the reactions selected from the group consisting of: (i) treating the compound of formula (I) with a hydroxide of an alkaline metal or an alkaline earth metal to obtain the corresponding salt; and (ii) protecting any hydroxyl group to obtain the corresponding -OC(O)-alkyl group.

12. Process for the preparation of a compound of general formula (I) as defined in claim 3, wherein R¹-R⁹ and n have the meaning given in claim 3, X is - (CH2)ₙ, Y=O and m=1, which comprises reacting:
a) a compound of formula (II) 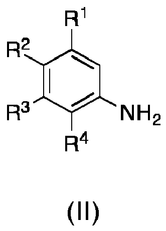 wherein R¹, R², R³ and R⁴ have the meaning given above;
with
b) a compound of general formula (III), 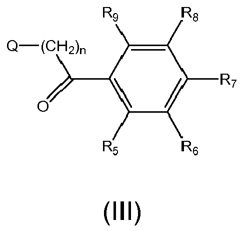 wherein Q can be a chlorine, bromine or iodine atom, or a leaving group, and R⁵, R⁶, R⁷, R⁸, R⁹ and n have the meaning given above;
in the presence of
c) a base, either organic or inorganic; and
d) a solvent
leaving the reaction to react for 3 hours at the most, and, optionally, performing one or more of the reactions selected from the group consisting of: (i) treating the compound of formula (I) with a hydroxide of an alkaline metal or an alkaline earth metal to obtain the corresponding salt; and (ii) protecting any hydroxyl group to obtain the corresponding -OC(O)-alkyl group.

13. Process for the preparation of a compound of general formula (I) as defined in claim 3, wherein R¹-R⁹ and n have the meaning given in claim 3, X is - CH=CH-, Y=N-OH and m=0, which comprises:
a) preparing a compound of general formula (I) as described in claim 11; and
b) further reacting the obtained product of formula (I) with a mixture of hydroxylamine hydrochloride and phenolphthalein in the presence of an excess of sodium methoxide in methanol.

14. Process for the preparation of a compound of general formula (I) as defined in claim 3, wherein R¹-R⁹ and n have the meaning given in claim 3, X is - (CH₂)ₙ, Y=N-OH and m=0, which comprises:
c) preparing a compound of general formula (I) as described in claim 12; and
d) further reacting the obtained product of formula (I) with a mixture of hydroxylamine hydrochloride and phenolphthalein in the presence of an excess of sodium methoxide in methanol.

15. A pharmaceutical composition that comprises at least a compound of formula (I) as defined in any of claims 3 to 10, or a pharmaceutically acceptable solvate or a salt or prodrug thereof, and at least a pharmaceutically acceptable excipient.
